# EUROPEAN PATENT SPECIFICATION

(11) **EP 2 852 361 B1**
(45) Date of publication and mention of the grant of the patent: **03.07.2019**
(21) Application number: 13793571.4
(22) Date of filing: 22.05.2013
(51) Int. Cl.: A61G 7/00, A61G 7/057, A61F 5/56

(54) **ADVERSE EVENT MITIGATION SYSTEMS, METHODS AND DEVICES**
SYSTEME, VERFAHREN UND VORRICHTUNGEN ZUR ABSCHWÄCHUNG UNERWÜNSCHTER EREIGNISSE
SYSTÈMES, PROCÉDÉS ET DISPOSITIFS D'ATTÉNUATION D'ÉVÈNEMENTS DOMMAGEABLES

(30) Priority: 22.05.2012 US 201261650022 P; 26.11.2012 US 201261729868 P; 15.03.2013 US 201361792911 P
(43) Date of publication of application: 01.04.2015
(73) Proprietor: Hill-Rom Services, Inc., Batesville, IN 47006 (US)
(72) Inventor: HOOD, Michael S., Batesville, Indiana 47006 (US); RIBBLE, David, Indianapolis, Indiana 46202 (US); GAZAGNES, Laetitia, F-34080 Montpellier (FR); DEGUIGNET, Pierre, F-34830 Clapiers (FR); BHAI, Aziz A., Fishers, Indiana 46038 (US); AGDEPPA, Eric D., Cincinnati, Ohio 45249 (US); WIGGERMANN, Neal, Batesville, Indiana 47006 (US); HOWELL, Charlie, Batesville, Indiana 47006 (US); SRIVASTAVA, Varad, Batesville, Indiana 47006 (US); PENNINGER, Jason, Indianapolis, Indiana 46220 (US)
(74) Representative: Findlay, Alice Rosemary
(86) International application number: PCT/US2013/042313
(87) International publication number: WO 2013/177338

(56) References cited:
- EP-A2- 0 674 893
- EP-A2- 2 494 946
- WO-A1-2013/166003
- KR-A- 20110 083 167
- US-A- 5 611 096
- US-A1- 2007 163 043
- US-A1- 2009 094 744
- US-A1- 2011 035 057
- US-A1- 2011 263 950
- US-A1- 2011 301 440
- US-A1- 2012 073 054

## Description

### BACKGROUND OF THE DISCLOSURE

This disclosure relates generally to adverse event mitigation systems, devices and methods. More particularly, but not exclusively, one illustrative embodiment relates to a system configured to initiate an intervention to help reduce the likelihood of an adverse event occurring and/or stop an adverse event in progress. While various systems have been developed, there is still room for improvement. Thus, a need persists for further contributions in this area of technology.

KR20110083167 discloses a bed which comprises a divided mattress which raise or lower the partial mattresses.

US 5611096 discloses a therapeutic bed with bladders which are alternately inflatable to rotate a mattress. Guard bladders may be provided to prevent a user from falling during rotation.

EP 2494946 discloses a method of positioning an occupant of a bed in a desired position.

### SUMMARY OF THE DISCLOSURE

The invention is defined by the claims.

Additional features alone or in combination with any other feature(s), including those listed above and those listed in the claims and those described in detail below, can comprise patentable subject matter. Others will become apparent to those skilled in the art upon consideration of the following detailed description of illustrative embodiments exemplifying the best mode of carrying out the invention as presently perceived.

### BRIEF DESCRIPTION OF THE DRAWINGS

Referring now to the illustrative examples in the drawings, wherein like numerals represent the same or similar elements throughout:
FIG. 1 is a partial diagrammatic view of an adverse event mitigation system according to one embodiment of the current disclosure;
FIG. 2 is a side perspective view of a person support apparatus and person support surface of Fig. 1;
FIG. 3 is a cross-sectional side view of the deck of the upper frame and the person support surface of Fig. 2;
FIG. 4 is a cross-sectional view of a portion of the person support surface of Fig. 2 showing the layers of the person support surface;
FIG. 5 is a side view of the person support surface of Fig. 2 according to another embodiment showing supports configured to support the cervical vertebrae and scapula;
FIG. 6 is a block diagram of a proactive procedure according to one embodiment;
FIG. 7 is a block diagram of a reactive procedure according to one embodiment;
FIG. 8 is a block diagram of a predictive procedure according to one embodiment; and
FIG. 9 is a partial diagrammatic view of a garment configured to rotate a person.

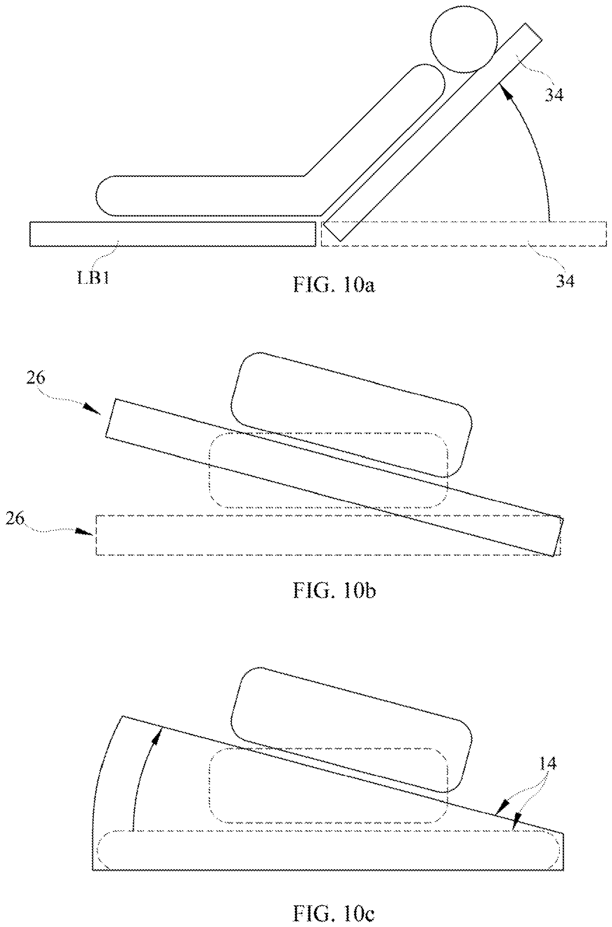

FIGS. 10A-10C are partial diagrammatic views of a person support apparatus configured to rotate a person in accordance with one or more embodiments disclosed herein.
FIG. 11 is a perspective view of an exemplary support system supporting a user on a sleep surface defined by the support system;
FIG. 12 is a perspective side view of the support system shown in FIG. 11;
FIG. 13 is a side elevational view of the support system shown in FIG. 11;
FIG. 14 is a front elevational view of the support system shown in FIG. 11;
FIG. 15 is a schematic view of an exemplary support system;
FIG. 16 is a partial front view of the support system shown in FIG. 15 illustrating a lateral rotation of planes;
FIG. 17 is a partial side view of the support system shown in FIG. 15 illustrating a longitudinal rotation of planes;
FIG. 18 shows an exemplary control system operatively coupled to the support system shown in FIG. 15;
FIG. 19 is a side view of an exemplary support system including a plurality of independently rotatable support pieces;
FIG. 20A is a cross-sectional view of the support system shown in FIG. 19 taken along sectional line 20A in FIG. 19;
FIG. 20B is a cross-sectional view of the support system shown in FIG. 19 taken along sectional line 20A in FIG. 19 and rotated 180° about a longitudinal axis of the support system;
FIG. 21 is side view of an exemplary support system including a plurality of support pieces;
FIG. 22 is a side view of the support system shown in FIG. 21 including a spacer positioned between adjacent support pieces;
FIG. 23 is a side view of an exemplary support system including a plurality of support pieces;
FIG. 24 is a side view of the support system shown in FIG. 23 including a spacer replacing one of the support pieces;
FIG. 25 is a perspective view of an exemplary support system showing a greater number of support wedges to allow more gradual changes in edge angle along a length of a sleep surface;
FIG. 26 is a top plan view of an exemplary support system supporting a user on a sleep surface defined by the support system;
FIG. 27 is a side view of the support system shown in FIG. 26 illustrating a continuous support piece exhibiting a gradual density transition along a longitudinal length of the sleep surface;
FIG. 28 is a top plan view of an exemplary dynamic support system;
FIG. 29 is a front view of an exemplary dynamic support system;
FIG. 30 is a top view of the dynamic support system shown in FIG. 29;
FIG. 31 is a side view of the dynamic support system shown in FIG. 29 illustrating a lateral rotation of planes;
FIGS. 32-35 illustrate various configurations of fluid bladders forming at least a portion of the dynamic support system shown in FIG. 29;
FIG. 36 is a schematic view of an exemplary dynamic support system;
FIG. 37 illustrates an exemplary method for monitoring sleep activities of a user positioned on a dynamic support system, such as the support system shown in FIG. 36;
FIGS. 38-41 illustrate an exemplary heuristic control of an apnea therapy surface function;
FIG. 42 is a perspective view of an exemplary continuous lateral rotation therapy (CLRT) system;
FIG. 43 is a front view of a control system configured to control the CLRT system shown in FIG. 42;
FIG. 44 is a cross-sectional view of the support system shown in FIG. 42;
FIG. 45 is a cross-sectional view of a portion of the support system shown in FIG. 42;
FIG. 46 is a cross-sectional view of an exemplary support system with fixed-length bands to restrict fluid bladder inflation;
FIG. 47 is a front perspective view of a front portion of an exemplary posture shirt; and
FIG. 48 is a rear view of a back portion of the posture shirt shown in FIG. 47.

### DETAILED DESCRIPTION OF THE DRAWINGS

While the present disclosure can take many different forms, for the purpose of promoting an understanding of the principles of the disclosure, reference will now be made to the embodiments illustrated in the drawings, and specific language will be used to describe the same. No limitation of the scope of the disclosure is thereby intended. Various alterations, further modifications of the described embodiments, and any further applications of the principles of the disclosure, as described herein, are contemplated.

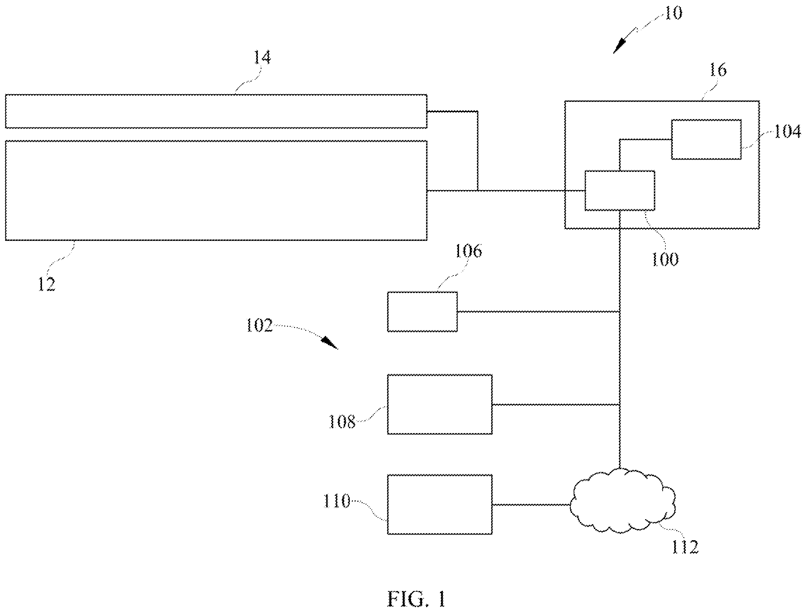

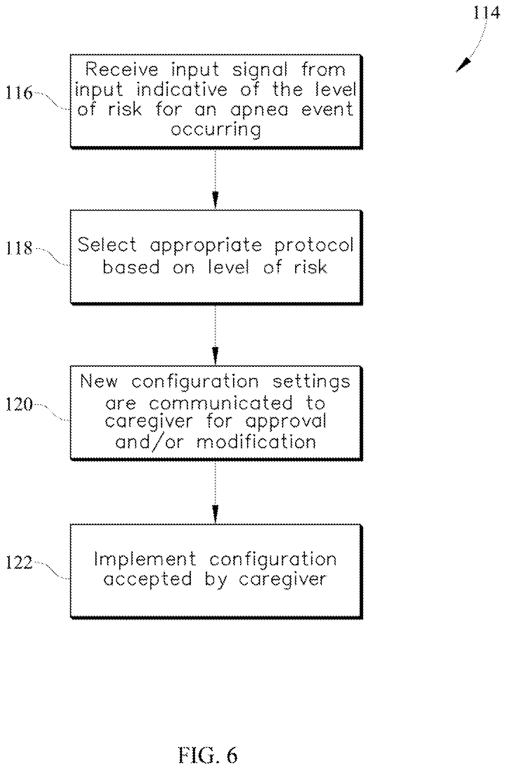

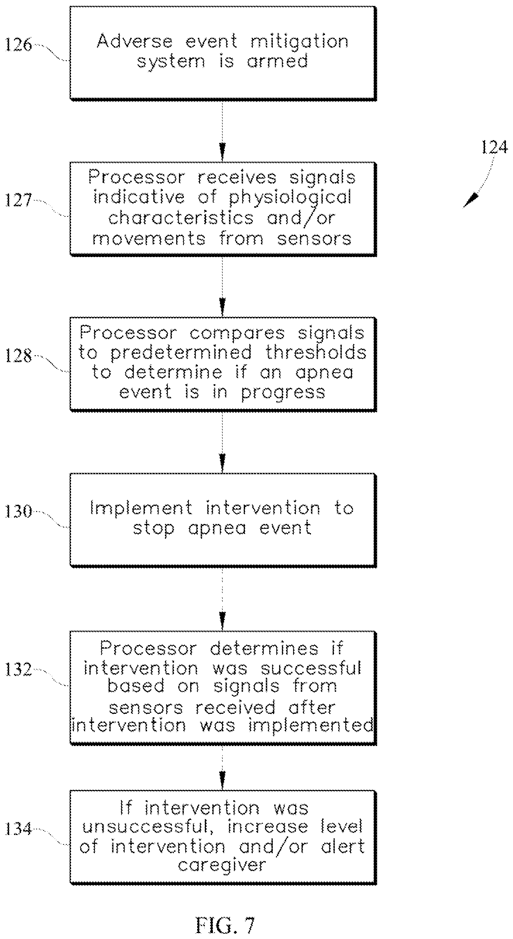

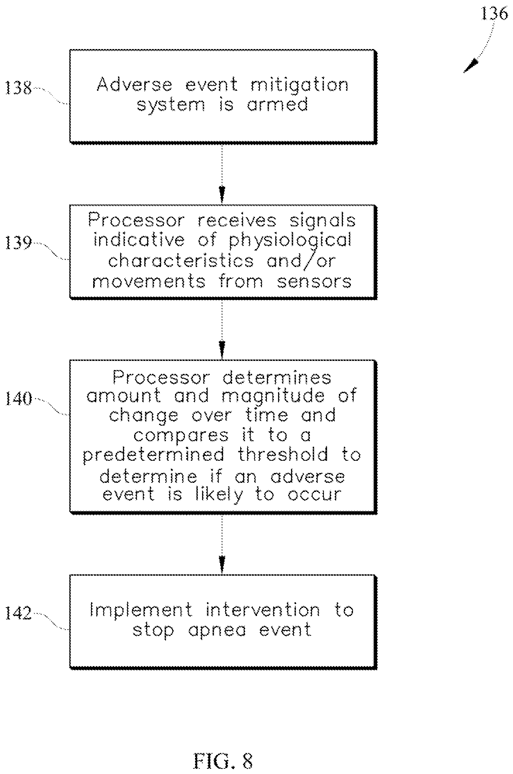

An adverse event mitigation system 10 according to one contemplated embodiment is shown in Figs. 1-8. The adverse event mitigation system 10 is configured to help reduce the likelihood of an adverse event occurring and/or stop an adverse event in progress. In some contemplated embodiments, the adverse event mitigation system 10 may help reduce the likelihood of obstructive sleep apnea occurring and/or may help stop an obstructive apnea event in progress. In other contemplated embodiments, the adverse event mitigation system 10 may help reduce the likelihood of other adverse events occurring and/or stop other adverse events in progress.

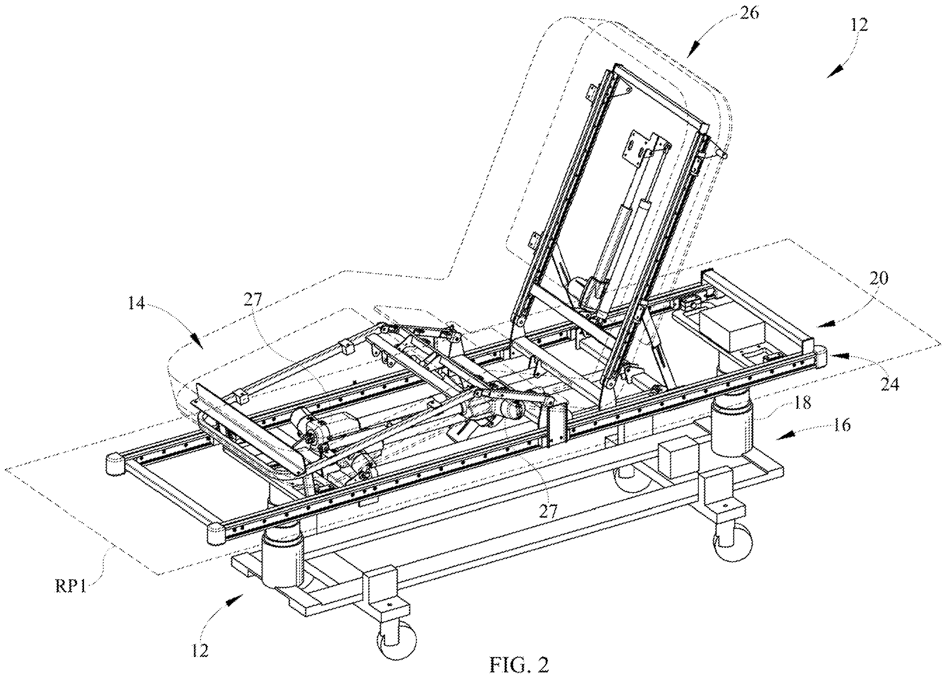

The adverse event mitigation system 10 includes a person support apparatus 12, a person support surface 14 supported on the person support apparatus 12, and a control system 16 as shown in Fig. 1. In some contemplated embodiments, the person support apparatus 12 is a hospital bed frame and the person support surface 14 is supported thereon as shown in Fig. 2. In other contemplated embodiments, the person support apparatus 12 can be a stretcher, an operating room table, or other person supporting structure. The person support apparatus 12 includes a lower frame 17, supports 18 or lift mechanisms 18 coupled to the lower frame 17, and an upper frame 20 movably supported above the lower frame 17 by the supports 18 as shown in Fig. 1. The lift mechanisms 18 are configured to raise and lower the upper frame 20 with respect to the lower frame 17 and move the upper frame 20 between various orientations, such as, Trendellenburg and reverse Trendellenburg.

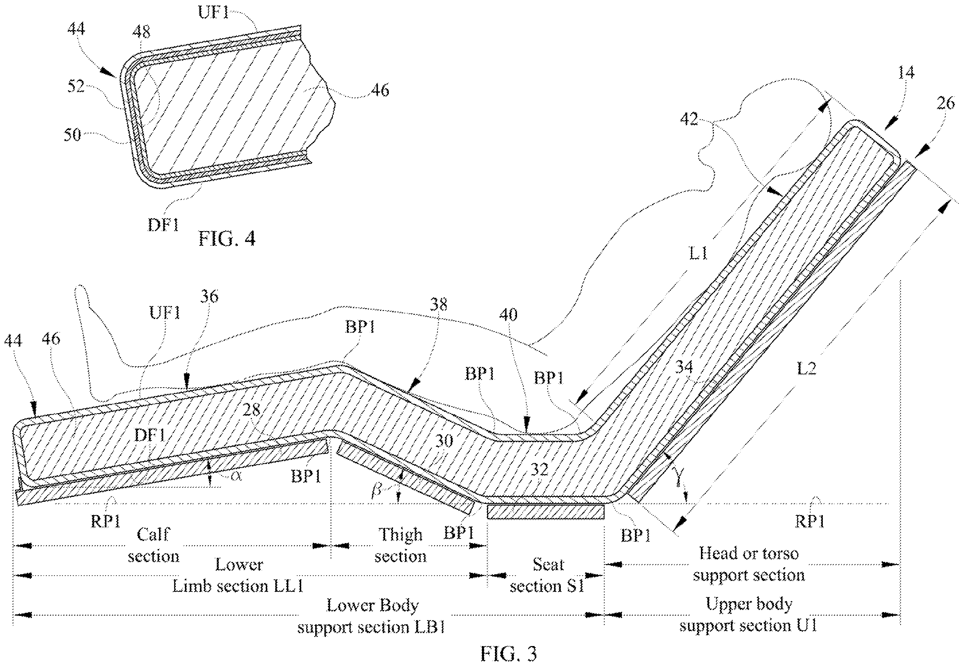

The upper frame 20 includes an upper frame base 24, a deck 26 coupled to the upper frame base 24, and a plurality of actuators 27 coupled to the upper frame base 24 and the deck 26 as shown in Fig. 2. The plurality of actuators 27 are configured to move at least a portion of the deck 26 along at least one of a longitudinal axis, which extends along the length of the upper frame 20, and a lateral axis, which extends across the width of the upper frame 20, between various articulated configurations with respect to the upper frame base 24. The deck 26 includes a calf section 28, a thigh section 30, a seat section 32, and a head and torso section 34 as shown in Fig. 3. The calf section 28 and the thigh section 30 define a lower limb support section LL1. The head and torso section 34 define an upper body support section U1. The seat section 32 defines the seat section S1. The calf section 28, the thigh section 30, and the seat section 32 define a lower body support section LB1. At least the calf section 28, the thigh section 30, and the head and torso section 34 are movable with respect to one another and/or the upper frame base 24. In some contemplated embodiments, the calf section 28, the thigh section 30, the seat section 32, and the head and torso section 34 cooperate to move the person support apparatus 12 between an substantially planar or lying down configuration and a chair configuration. In some contemplated embodiments, the calf section 28, the thigh section 30, the seat section 32, and the head and torso section 34 cooperate to move the person support apparatus 12 between a substantially planar or lying down configuration and an angled or reclined configuration. In some contemplated embodiments, the head and torso section 34 is moved such that it is at an angle of at least about 30° with respect to a reference plane RP1 passing through the upper frame 20.

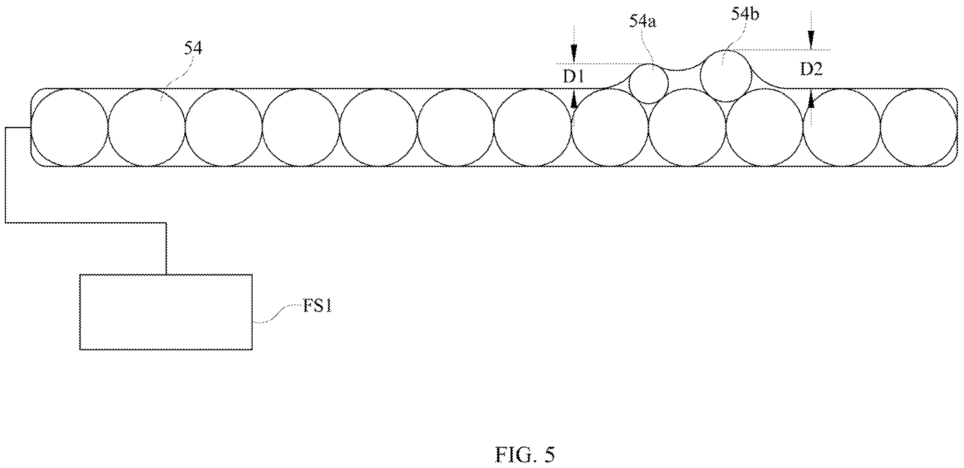

The person support surface 14 is configured to support a person thereon and move with the deck 20 between the various configurations. In some contemplated embodiments, the person support surface 14 is a hospital bed mattress as shown in Fig. 2-4. In some contemplated embodiments, the person support surface 14 is a consumer mattress. In some contemplated embodiments, the person support surface 14 includes a heat and moisture regulating topper positioned on the person support surface 14. In some contemplated embodiments, the person support surface 14 can include a pressure mapping topper positioned on the person support surface 14. The person support surface 14 includes a calf portion 36, a thigh portion 38, a seat portion 40, and a head and torso portion 42 as shown in Fig. 3, which is supported on corresponding sections of the deck 26. In one illustrative embodiment, the deck sections help move and/or maintain the various portions of the person support surface 14 at angles α, β and γ with respect to the reference plane RP1. In some contemplated embodiments, the person support surface 14 is a non-powered (static) surface. In some contemplated embodiments, the person support surface 14 is a powered (dynamic) surface configured to receive fluid from a fluid supply FS1 as shown in Fig. 5.

The person support surface 14 includes a mattress cover 44 and a mattress core 46 as shown in Figs. 3 and 4. In some contemplated embodiments, the person support surface 14 includes a temperature and moisture regulating topper (not shown) coupled to the mattress cover 44. The mattress cover 44 encloses the mattress core 46 and includes a fire barrier 48, a bottom ticking 50 or durable layer 50, and a top ticking 52. In some contemplated embodiments, the fire barrier 48 is the innermost layer of the cover 44, the top ticking 52 is the outermost layer, and the bottom ticking 50 is positioned between the fire barrier 48 and the top ticking 52 and is not coupled to the top ticking 52. The bottom ticking 50 and the top ticking 52 are vapor and air impermeable. In some contemplated embodiments, the top ticking 52 and the bottom ticking 50 are composed of polyurethane coated nylon and the bottom ticking 50 is configured to facilitate movement of the top ticking 52 with respect to the fire barrier 48. In other contemplated embodiments, the top ticking 52 and/or the bottom ticking 50 can be air and/or moisture permeable.

The mattress core 46 can be composed of a single type of material or a combination of materials and/or devices. In the case of a powered surface, the mattress core 46 includes at least one fluid bladder 54 therein that receives fluid from a fluid supply (not shown) to maintain the fluid pressure within the fluid bladder 54 at a predetermined level. In some contemplated embodiments, the powered surface can include non-powered components, such as, a foam frame that at least one fluid bladder 54 is positioned between. In some contemplated embodiments, a fluid bladder 54 can be positioned proximate to the thigh section and inflated or the calf portion 36, thigh portion 38, and/or seat portion 40 (including their corresponding deck sections) can be articulated to help prevent the occupant from sliding down the person support surface 14 as, for example, the inclination of the head and torso section 34 increases with respect to the reference plane RP1. In some contemplated embodiments, wedge shaped bladders are mirrored laterally about the centerline of the person support surface 14 and are configured to be inflated consecutively to laterally tilt the occupant, thereby relieving pressure on various portions of the occupant's body to help reduce the occurrences of pressure ulcers.

In some contemplated embodiments, the mattress core 46 includes inflatable fluid bladders 54a and 54b, which are configured to protrude from the patient facing surface of the person support surface 14 by at least about 70 mm (adjusted for pillow height) and about 20 mm to about 30 mm to support the cervical vertebrae and scapula, respectively. In some contemplated embodiments, the inflatable fluid bladders 54a and 54b are replaced foam bolsters or static air bladders or a combination thereof. In some contemplated embodiments, the distance the fluid bladders 54a and 54b protrude from the patient facing surface of the person support surface 14 can vary depending on any number of factors, including, but not limited to, a person's body type and the angle at which the surface is at with respect to the reference plane RP1. In some contemplated embodiments, the fluid bladders 54a and 54b can also be configured to laterally tilt the head and/or torso of the occupant. In some contemplated embodiments, wedge shaped fluid bladders (not shown) are positioned in the head and torso portion 42 and are configured to increase the angle of the occupant contacting surface of the head and torso portion 42 with respect to the seat portion 40 when inflated.

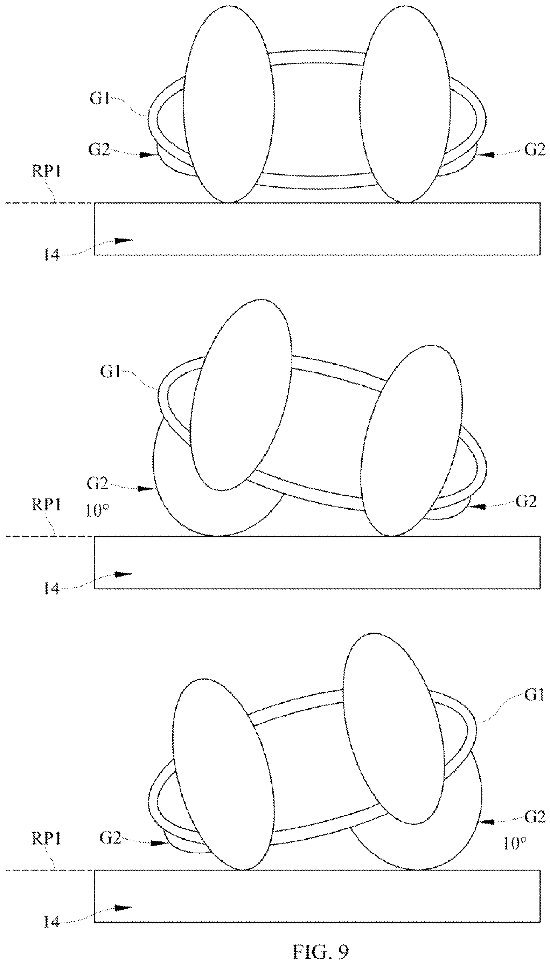

In some contemplated embodiments, the head and torso of the occupant can be tilted at different angles. For example, the person support apparatus 12 and/or the person support surface 14 can laterally rotate the occupant so that the torso is at an angle of about 10° with respect to the reference plane RP1 and the head is at an angle of about 180° with respect to the reference plane RP1. Rotation of the occupant's torso can help an occupant maintain their head at an angle of about 180° with respect to the reference plane RP1. In some contemplated embodiments, the person support surface 14 is configured to allow the occupant's body to be immersed into the surface to improve comfort with lateral positioning. In some contemplated embodiments, support blocks (not shown) can be placed on the surface 14 adjacent to the occupant to help maintain the position of the occupant. In some contemplated embodiments, the person support apparatus 12 and/or person support surface 14 can laterally rotate the occupant so that the torso is at an angle of about 10° with respect to the reference plane RP1 and fluid bladders 54a and 54b can rotate the occupant's head so that it is at an angle of at least about 180°. In some contemplated embodiments, the occupant can be wearing a garment G1 with fluid bladders G2 configured to be inflated to help laterally rotate the occupant so that the torso is at an angle of at least 10% with respect to the reference plane RP1 as shown in Fig. 9. In some contemplated embodiments, the garment G1 is configured to provide therapy, including, for example, percussion, vibration, and compression therapies. In some contemplated embodiments, the garment G1 is an airway clearance vest, such as the Vest® Airway Clearance System sold by Hill-Rom. In other contemplated embodiments, the garment G1 can be other therapy garments, including sequential compression devices (SCD). In some contemplated embodiments, fluid can be supplied to the garment G1 via the fluid supply FS1 configured to supply fluid to the fluid bladders 54a and 54b. In some contemplated embodiments, fluid is supplied to the garment G1 and/or fluid bladders G2 by a dedicated fluid supply (not shown). The angle of the occupant's head with respect to the reference plane RP1 may vary depending on the occupant's preferences, their risk of the adverse condition, or other factors.

In the case of a non-powered surface, the mattress core 46 is composed of a cellular engineered material, such as, single density foam. In some contemplated embodiments, the mattress core 46 includes at least one bladder 54, such as, a static air bladder or a static air bladder with foam contained there within, a metal spring and/or other non-powered support elements or combinations thereof. In some contemplated embodiments, the mattress core 46 includes multiple zones with different support characteristics configured to enhance pressure redistribution as a function of the proportional differences of a person's body. Also, in some embodiments, the mattress core 46 includes various layers and/or sections of foam having different impression load deflection (ILD) characteristics, such as, in the NP100 Prevention Surface, AccuMax Quantum™ VPC Therapy Surface, and NP200 Wound Surfaces sold by Hill-Rom®.

The control system 16 is configured to change at least one characteristic of the person support apparatus 12 and/or person support surface 14 to help reduce the likelihood of an adverse event occurring and/or stop an adverse event in progress. The control system 16 includes a processor 100, an input 102, and memory 104. In some contemplated embodiments, the input 102 includes a sensor 106, such as, a position sensor, a pressure sensor, a temperature sensor, an acoustic sensor, and/or a moisture sensor, configured to provide an input signal to the processor 100 indicative of a physiological characteristic of the occupant, such as, the occupant's heart rate, respiration rate, respiration amplitude, skin temperature, weight, and position. In some contemplated embodiments, the sensors 106 are incorporated into the person support surface 14 or topper positioned on the person support surface, for example, as disclosed in U.S. Patent No. 7,515,059 to Price et al. and U.S. Patent Publication No. 2011/0068928 to Riley et al. In some contemplated embodiments, the sensors 106 include, for example, RFID tags, accelerometers, proximity sensors, level sensors, or other physical tracking sensors that may be integrated into or coupled to, for example, ear plugs, ear phones, adhesive sensors, earlobe clips, eye covers, hats, nose strips or other devices that are attached to the patient's head or worn by the patient so that the position/orientation of the patient's head can be tracked. Information captured by monitoring the lateral position of the user's upper respiratory tract has several benefits, including one or more of the following: providing more accurate measurements of the upper respiratory angle for diagnosis of positional obstructive sleep apnea (in one example, sleep labs can use the information to more accurately diagnose POSA); providing biofeedback to help the user to train to maintain a posture that prevents POSA; tracking performance of the system to determine if the system is achieving a sufficient upper respiratory angle to prevent apnea; monitoring compliance to determine if the system is being used; monitoring the upper respiratory angle and recording the angle when a sleep apnea event occurs; and controlling a surface capable of providing lateral rotation as a function of the inputs from the sensors 106, tracking whether optimal lateral position has been achieved, and controlling the system to achieve a desired head lateral position and/or upper respiratory angle. In some contemplated embodiments, the sensors 106 are tracked by reading devices (i.e., an RFID reader) in a siderail, person support surface, deck, headboard, or location on or in the person support apparatus 10 or person support surface 14, or on or in a headwall in the room or other location in the room. In some contemplated embodiments, the sensor 106 includes a camera positioned at the foot of the bed or above the bed, as disclosed in U.S. Patent Publication No. 2012/0029879 to Sing et al., for example, to track the orientation of the person's head.

In some contemplated embodiments, the input 102 includes a user interface 108 configured to receive information from a caregiver or other user. In other contemplated embodiments, the input 102 is an Electronic Medical Record (EMR) system 110 in communication with the processor 100 via a hospital network 112. In some contemplated embodiments, the processor 100 can output information, automatically or manually upon caregiver input, to the EMR for charting, which can include therapy initiation and termination, adverse event occurrence information, therapy protocol used, caregiver ID, and any other information associated with the occupant, caregiver, person support apparatus 12, person support surface 14, and adverse event.

The memory 104 stores one or more instruction sets configured to be executed by the processor 100. The instruction sets define procedures 114 that, when executed by the processor, cause the processor 100 to implement one or more protocols that modify the configuration of the person support apparatus 12 and/or the person support surface 14. In one illustrative embodiment, the instruction set defines a proactive procedure 114 that causes the processor 100 to configure the person support apparatus 12 and/or the person support surface 14 in response to an input specifying that the occupant is at risk for sleep apnea. Procedure 114 begins with step 116 in which the processor 100 receives an input signal from the input 102 indicative of the level of risk for an apnea event occurring. In some contemplated embodiments, the level of risk is input from a field in the occupant's EMR. In some contemplated embodiments, the level of risk is input by a caregiver through the user interface 108, which may arise from a doctor's order or be based on a patient scoring system. In some contemplated embodiments, the level of risk is determined based on a risk score that is calculated by the processor 100 based on a number of factors, including, but not limited to, those listed in the table below:

| | |
|---|---|
| Predisposing physical characteristics | • BMI in the 95^{th} percentile for age and gender (i.e., 35 kg/m² ) |
| | • 17 inch neck circumference for men (16 inches for women) |
| | • craniofacial abnormalities that affect the airway |
| | • anatomical nasal obstructions |
| | • tonsils that nearly touch or do touch in the medline |
| History of apparent airway obstruction during sleep | • loud or frequent snoring |
| | • observed pauses in breathing while asleep |
| | • awakening from sleep with a choking sensation |
| | • frequent arousal from sleep |
| Somnolence | • frequent somnolence or fatigue despite getting an adequate amount of sleep |
| | • falling asleep easily in a nonstimulating place despite adequate sleep |

| Sleep study results | |
|---|---|
| Invasiveness of surgery and anesthesia | • superficial under local or peripheral nerve block without sedation |
| | • superficial with moderate sedation or general anesthesia |
| | • peripheral with spinal or epidural anesthesia and no more than moderate sedation |
| | • peripheral with general anesthesia |
| | • airway surgery with moderate sedation |
| | • major surgery with general anesthesia |
| | • airway surgery with general anesthesia |
| Requirement of postoperative opioids | • none |
| | • low-dose oral opioids |
| | • high-dose oral opioids or neuraxial or parenteral opioids |

| Estimation of perioperative risk | |
|---|---|
| Sex and age of occupant | • Estimated sleep disordered breathing is 9% in women and 24% in men with the prevalence for obstructive sleep apnea being 2% in women and 4% in men. The percentages likely increase for older and more obese people |

In circumstances where an occupant is known to snore frequently, has a high BMI, has had major surgery, and/or requires postoperative opioids, the occupant may have an elevated risk. In circumstances where an occupant has a low BMI, is not known to snore, had superficial surgery, and/or does not require postoperative opioids, the occupant may have a reduced risk. An example of a scoring system is shown in the table below, where a score of 4 can indicate an increased risk, and a score of greater than 4 can indicate a significantly increased risk.

| | | |
|---|---|---|
| I. Severity of apnea | • Based on sleep study | 0-3 points |
| | • Based on predisposing physical characteristics, history of apparent airway obstruction during sleep, and somnolence | |
| | ∘ None | 0 points |
| | ∘ Mild | 1 point |
| | ∘ Moderate | 2 points |
| | ∘ Severe | 3 points |
| II. Surgery and Anesthesia | • Invasiveness | 0-3 points |
| | • Type | |
| | ∘ Superficial under local or peripheral nerve block without sedation | 0 points |
| | ∘ Superficial with moderate sedation or general anesthesia | 1 point |
| | ∘ Peripheral with spinal or epidural anesthesia and no more than moderate sedation | 1 point |
| | ∘ Peripheral with general anesthesia | 2 points |
| | ∘ Airway surgery with moderate sedation | 2 points |
| | ∘ Major surgery with general anesthesia | 3 points |
| | ∘ Airway surgery with general anesthesia | 3 points |
| III. Postoperative opioids | • Requirement | 0-3 points |
| | • Dosage | |
| | ∘ None | 0 points |
| | ∘ Low-dose oral | 1 point |
| | ∘ High dose oral, parenteral or neuraxial | 3 points |
| Risk = Score from I + Score from the greater of II and III (0-6 points) | | |

In step 118, the processor 100 determines which protocol should be implemented based on the level of risk. One type of the protocol is a default protocol set according to the hospital's standard operating procedures/guidelines for patients with specific risk profiles. Another type of protocol is a variable protocol that modifies the default protocol based on the occupant's preferences (i.e., prefers to sleep on their left side), the caregiver's observations, and/or information about the occupant's medical condition (i.e., pressure ulcer susceptibility, BMI, type of surgery, etc.) from the occupant's EMR, sensors 106, and/or other input 102. In some contemplated embodiments, the protocol can be modified to exclude or limit a therapy or movement. For example, the protocol can be prevented from increasing the head of bed angle (the angle between the reference plane RP1 and the head and torso section 34 or head and torso portion 42) above a predetermined threshold where the occupant is recovering from abdominal surgery. In some contemplated embodiments, the protocol can caution the caregiver against implementing the configurations based on information obtained from the occupant's EMR or other sources.

In step 120, once the protocol is selected, the configuration settings are communicated to the caregiver, for example, on a graphical user interface or other display device, and the caregiver is prompted to accept/modify the settings. In some contemplated embodiments, the configuration settings can be communicated to a hand held device. In one example, the protocol may require the head of bed angle to be greater than about 35° and the lateral tilt angle to be greater than 15° with respect to the reference plane RP1 for an occupant with an elevated risk score. In another example, the protocol may require the head of bed angle to be about 10° to about 15° and the lateral tilt angle to be about 10° to about 15° with respect to the reference plane RP1 for an occupant with a reduced risk score. In some contemplated embodiments, the upper frame 20 can also be moved to a Trendellenburg or reverse Trendellenburg orientation. In some contemplated embodiments, the protocol can require additional therapies to be active, such as, continuous lateral rotation where, for example, the lateral tilt angle changes every 30-120 minutes depending on the occupant's risk of developing pressure ulcers. In some contemplated embodiments, the sleep stage of the occupant can be taken into account so that the occupant is moved only when they are in a sleep state that would allow them to be moved without waking up. In some contemplated embodiments, the person support apparatus 12 and/or the person support surface 14 are returned to the configuration they were in prior to the implementation of the protocol before the occupant wakes up. In some contemplated embodiments, a manual stop button can be included so that the caregiver, occupant, or other person can terminate the protocol in the event of an emergency. In some contemplated embodiments, the protocol can automatically be terminated when an emergency condition occurs, such as, when the CPR handle (not shown) is pulled by a caregiver or the occupant is coding. In some contemplated embodiments, the procedure 114 can be terminated remotely by a caregiver, such as, via the hospital network or over a nurse call system.

In some contemplated embodiments, the position and/or the orientation of the occupant with respect to patient facing surface of the person support surface 14 is detected and can influence how the person support surface 14 and/or the person support apparatus 12 are configured to move the occupant to the desired position. For example, if the occupant is positioned along the left edge of the patient facing surface of the person support surface 14, the protocol will not rotate them to the left. In some contemplated embodiments, the protocol is terminated because the occupant is in the correct position. In some contemplated embodiments, the protocol helps to maintain the occupant in the position. The position of the occupant on the person support surface 14 can be determined a number of ways, including sensing the force distribution on the upper frame 20 utilizing one or more load cells (not shown) coupled to the upper frame 20, calculating the occupant's center of gravity using the one or more load cells, sensing pressures within the fluid bladders 54, using a camera (not shown) or 3D sensor (not shown), or using other methods.

In step 122, if the caregiver accepts the configuration or changes the configuration and accepts the new configuration, the processor 100 implements the configuration for a predetermined time. In some contemplated embodiments, the processor 100 can implement the configuration the moment the caregiver approves it and stop or change the configuration when the caregiver deactivates it. In some contemplated embodiments, the processor 100 will wait to implement the configuration until the occupant is in a predetermined sleep stage and will return to the initial configuration when the occupant begins to wake up. In some contemplated embodiments, procedure 114 does not require the caregiver to confirm or accept the settings, and instead automatically initiates the configuration. For example, the configuration can be automatically initiated a predetermined time after the occupant departed from the surgical room, which can be determined based on the occupant's EMR. In some contemplated embodiments, the configuration will not be implemented if the bed is unoccupied.

Procedure 114 can be used for a number of other adverse conditions. In some contemplated embodiments, procedure 114 can be used to determine if a person is at risk for or has gastroesophageal reflux disease and select a protocol that assists the occupant in maintaining a left lateral decubitus position or semi-reclining position while sleeping. In some contemplated embodiments, procedure 114 can be used to determine if a person is at risk for or has chronic respiratory insufficiency and select a protocol for the caregiver to approve that assists the occupant in maintaining a left lateral decubitus position while sleeping. In some contemplated embodiments, the procedure can be used to determine if a person is at risk for of has allergies to, for example, feather or down filled pillows, cushions or covers, and can alert the caregiver so that they can remove the item. In other contemplated embodiments, procedure 114 can be used to determine if the person is at risk for or has one or more other conditions, such as, for example, asthma, pregnancy, sleep paralysis or hallucinations, snoring, stroke bruxism, coughing, hypoxaemia in geriatric inpatients, stroke, or tuberculosis, that might be affected negatively by sleeping in the supine position and select a protocol and/or alert the caregiver so that the person support apparatus 12 and/or the person support surface 14 can be configured to maintain the occupant in a desirable position. In some contemplated embodiments, the procedure 114 can be used to change the sleeping position of occupants to help stimulate blood oxygenation, which can undesirably decrease as the occupant remains stationary.

In another illustrative embodiment, the instruction set causes the processor 100 to carry out a responsive procedure 114 that configures the person support apparatus 12 and/or the person support surface 14 in response to detection of an adverse event, such as, an apnea event. Procedure 124 begins with step 126 where the adverse event mitigation system is armed manually by the caregiver or automatically based on information from the occupant's EMR, the caregiver, or calculated by the processor 100.

In step 127, the processor 100 receives signals from the sensors 106 indicative of the physiological characteristics of the occupant, including, but not limited to, the occupant's heart rate and the respiration characteristics, such as, amplitude and rate, and/or the amount of movement of the occupant.

In step 128, the processor 100 compares the signals from the sensors 106 to predetermined thresholds to determine if an apnea event is in progress. For example, if there is an interval of at least about 10 seconds between breaths then the person is likely having an apnea event. In another example, if the person is taking less than about 25% of a normal breath for at least about 10 seconds, then the person is likely having an apnea event In another example, if there is a drop in oxygen saturation of at least about 4%, then the person is likely having an apnea event. If the person is taking between about 26% and about 69% of a normal breath, the person is likely having a hypopnea event. In some contemplated embodiments, the processor 100 determines that an adverse event is in progress and alerts the caregiver that an adverse event is occurring and that it is likely not an apnea event based on the position of the occupant and/or the configuration of the person support apparatus 12 and/or the person support surface 14, the occupant's risk score, and/or the occupant's physiological characteristics, medical information from the occupant's EMR, and/or other information. In some contemplated embodiments, the caregiver can be alerted by a visual or audible alarm on the person support apparatus 12, a visual or audible alarm located in the room where the person support apparatus 12 is located, and/or a visual or audible alarm located proximate to the room, such as, in the hall way.

In some contemplated embodiments, the caregiver can be notified remotely by a communication system (not shown). In some contemplated embodiments, the communication system is a patient/nurse call system that can include patient stations capable of generating hospital calls and a remote master station which can prioritize and store the calls. One example of such a system is disclosed in U.S. Patent No. 5,561,412 issued on October 1, 1996 to Novak et al., which is incorporated by reference herein in its entirety. Another example of such a system is disclosed in U.S. Patent No. 4,967,195 issued on May 8, 2006 to Shipley, which is incorporated by reference herein in its entirety.

In another contemplated embodiment, the communication system is a system for transmitting voice and data in packets over a network with any suitable number of intra-room networks that can couple a number of data devices to an audio station, where the audio station couples the respective intra-room network to a packet based network. One example of such a system is disclosed in U.S. Patent No. 7,315,535 issued on January 1, 2008 to Schuman, which is incorporated by reference herein in its entirety. Another example of such a system is disclosed in U.S. Patent Publication No. 2008/0095156 issued on April 24, 2008 to Schuman, which is incorporated by reference herein in its entirety.

In yet another contemplated embodiment, the communication system is includes a patient/nurse call system, a nurse call/locating badge, an electronic medical record (EMR) database, and one or more computers programmed with work-flow process software. One example of such a system is disclosed in U.S. Patent Publication No. 2008/0094207 published on Apr. 24, 2008 to Collins, Jr. et al., which is incorporated by reference herein in its entirety. Another example of such a system is disclosed in U.S. Patent Publication No. 2007/0210917 published on September 13, 2007 to Collins, Jr. et al., which is incorporated by reference herein in its entirety. Yet another example of such a system is disclosed in U.S. Patent No. 7,319,386 published on January 15, 2008 to Collins, Jr. et al., which is incorporated by reference herein in its entirety. It should be appreciated that the work-flow process software can be the NaviCare® software available from Hill-Rom Company, Inc. It should also be appreciated that the work-flow process software can be the system disclosed in U.S. Patent No. 7,443,303 issued on October 28, 2008 to Spear et al., which is incorporated by reference herein in its entirety. It should further be appreciated that the badge can be of the type available as part of the ComLinx™ system from Hill-Rom Company, Inc. It should also be appreciated that the badge can also be of the type available from Vocera Communications, Inc.

In still another contemplated embodiment, the communication system is configured to organize, store, maintain and facilitate retrieval of bed status information, along with the various non-bed calls placed in a hospital wing or ward, and remotely identify and monitor the status and location of the person support apparatus, patients, and caregivers. One example of such a system is disclosed in U.S. Patent No. 7,242,308 issued on July 10, 2007 to Ulrich et al., which is incorporated by reference herein in its entirety. It should be appreciated that the remote status and location monitoring can be the system disclosed in U.S. Patent No. 7,242,306 issued on July 10, 2007 to Wildman et al., which is incorporated by reference herein in its entirety. It should also be appreciated that the remote status and location monitoring can be the system disclosed in U.S. Patent Publication No. 2007/0247316 published on October 25, 2007 to Wildman et al., which is incorporated by reference herein in its entirety.

In step 130, if the processor determines art apnea event is in progress, the processor 100 configures the person support surface 14 and/or the person support apparatus 12 to intervene and help stop the apnea event. In one illustrative embodiment, the processor 100 inflates a bladder 54 in the person support surface 14 to rotate the occupant onto their side such that they are at an angle of about 10° with respect to the reference plane RP1. In some contemplated embodiments, the upper frame 20 can be rotated along the longitudinal axis to laterally tilt the occupant. In another illustrative embodiment, the processor 100 increases the head of bed angle to about 15° by moving the head and torso section 34 of the person support apparatus 12 and/or inflating a bladder 54 in the person support surface 14. In some contemplated embodiments, the processor 100 increases the head of bed angle and laterally rotates at least a portion of the occupant's body. In some contemplated embodiments, the processor 100 implements additional therapies, such as, for example, continuous lateral rotation therapy (CLRT), percussion vibration therapy, heat and moisture management therapy, rotation therapy, or other therapies depending on the occupant's risk for developing additional adverse conditions, such as, pressure ulcers.

In some contemplated embodiments, procedure 124 includes step 132 and step 134 in which the processor 100, after implementing the intervention, receives signals from the sensors 106 indicative of the occupant's physiological characteristics and/or the amount of movement of the occupant, and compares them with the predetermined thresholds to determine if the intervention was successful and the apnea event has ceased. In one illustrative embodiment, the processor 100 waits a predetermined amount of time, such as, 5 seconds, after the intervention has been implemented before it receives signals from the sensors 106. In some contemplated embodiments, the processor 100 can receive signals from the sensors 106 as the intervention is implemented and stop intervening or maintain the current level of intervention when the apnea event has ceased. For example, the processor 100 receives signals from the sensors 106 as the head of bed angle and/or the lateral tilt angle are gradually increased and stops increasing the head of bed angle and/or the lateral tilt angle once the apnea event has ceased. In some embodiments, the head of bed angle and/or the lateral tilt angle are gradually increased and an alarm is activated when the angle reaches a predetermined threshold. If the processor 100 determines that the intervention was successful, the processor 100 can cause the person support apparatus 12 and/or the person support surface 14 to maintain the current configuration or cause it to return to its initial position.

If the processor 100 determines that the apnea event is still in progress, the processor 100 can increase the level of intervention. In one illustrative embodiment, the head of bed angle and/or the lateral tilt angle can be increased air additional 5°. In other embodiments, the stimuli can include vibration, sound, temperature, smells, lights (flashing and/or constant), or other stimulus or combinations thereof that may or may not wake the person. In some instances, the goal of the intervention is to stop the apnea event without waking the occupant up, which can include moving the person while the person is in a particular sleep stage and/or causing the person to move from a deeper sleep stage to a lighter sleep stage. In some contemplated embodiments, movement of the occupant can cease if the processor 100 detects the person is waking up (based on increased heart rate, respiration rate, and/or movement) or is moving to a lighter sleep stage. If the increased levels of intervention continue to be unsuccessful then the processor 100 can initiate an alarm on or near the person support apparatus 12 to wake the occupant and/or notify a caregiver via nurse call or other means of communication that they need to intervene. In some contemplated embodiments, if the processor 100 receives information that the occupant is sedated, the processor 100 can move the occupant to a position, such as, for example, a sitting position or chair position.

In another illustrative embodiment, the instruction set causes the processor 100 to carry out a proactive procedure 136 that configures the person support apparatus 12 and or the person support surface 14 when the processor 100 predicts the onset of an adverse event. Procedure 136 begins with step 138 where the system for mitigating adverse conditions is armed by the caregiver or the bed or EMR based on the occupant's risk profile.

In step 139, the processor 100 receives signals from the sensors 106 indicative of the physiological characteristics of the occupant and/or the amount of movement of the occupant.

In step 140, the processor 100 stores the signal values in the memory 104 and determines an amount and/or a magnitude of change in the values for a predetermined time period. The processor 100 then compares the amount and/or the magnitude of change to a predetermined threshold to determine if an adverse event is likely to occur. In some contemplated embodiments, the processor 100 considers other factors, such as, the occupant's risk score, body position or orientation, person support apparatus 12 and/or person support surface 14 configurations, medical conditions, and/or other information from the caregiver, occupant's EMR, sensors 106, and/or person support apparatus 12 and/or person support surface 14 when determining the likelihood of an adverse event occurring. For example, if an occupant is at a high risk for apnea, is in the supine position, and the occupants respiration rate is decreasing, then an apnea event may occur. In another example, if an occupant's respiration amplitude decreases and the occupant's oxygen saturation decreases then an apnea event may occur. In another example, if an occupant's snoring is very loud and the occupant is at a high risk for apnea, an apnea event may occur. In another example, if an occupant is at high risk and the occupant is receiving 90% normal breath, an apnea event may be unlikely.

In some contemplated embodiments, prediction of an apnea event can be accomplished using a time-domain model of nonlinear time-lagged interactions between heart rate, respiration, and oxygen saturation to help determine when an apnea event is likely. In some contemplated embodiments, prediction of an apnea event can be accomplished using a Bayesian "belief network" model. In some contemplated embodiments, prediction of an apnea event can be accomplished using large memory storage and retrieval (LAMSTAR) artificial neural networks to analyze signals indicative of heart rate variability, nasal pressure, oronasal temperature, submental EMG, and electrooculography. In some contemplated embodiments, prediction of an apnea event can be accomplished by analyzing tracheal breath sounds.

In step 142, the processor configures the person support surface 14 and/or the person support apparatus 12 as previously described above with respect to procedure 114 and procedure 124 to intervene and help prevent the apnea event.

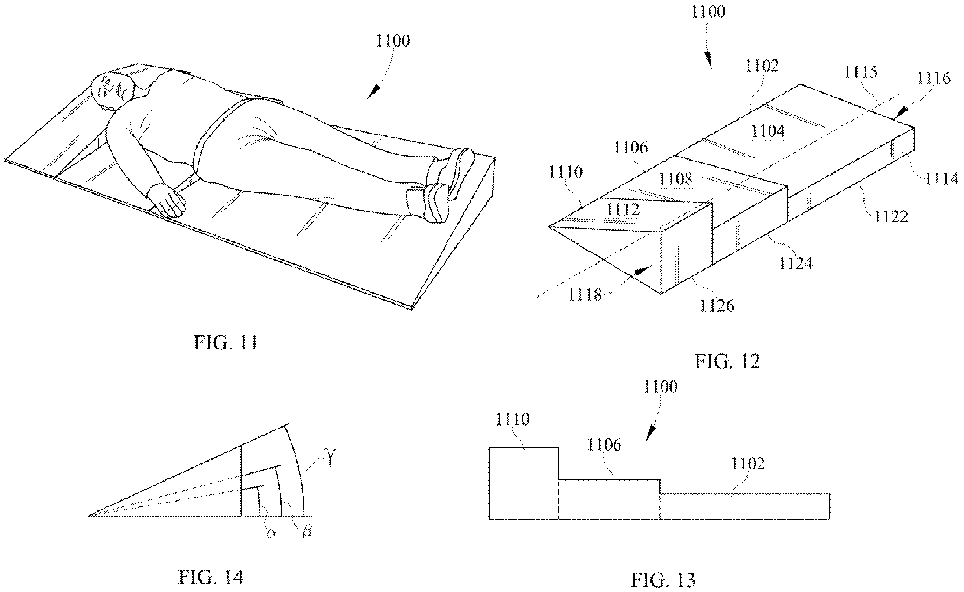

In another contemplated embodiment, retelling to FIGS. 11-18, a support system includes one or more support pieces or units that form a lateral support plane to prevent or restrict the user from sleeping in a supine position, and, more specifically, reduce a time duration that the user sleeps with his/her upper respiratory tract oriented vertically or at an undesirable lateral rotational angle with respect to a vertical plane substantially perpendicular to a horizontal plane. In certain embodiments, the lateral rotational angle of the user's head with respect to the vertical plane is at least 30 degrees and, more specifically, at least 45 degrees. In an alternative embodiment, the lateral rotational angle of the user's head with respect to the vertical plane may be less than 30 degrees. In one embodiment, the support pieces provide multiple support planes for supporting the user's body.

In one embodiment as shown in FIGS. 11-18, a support system 1100 suitable for supporting a user, such as a person, for example, includes plurality of support pieces, namely a first or leg support piece 1102 forming a first support plane 1104, a second or torso support piece 1106 forming a second support plane 1108, and a third or head support piece 1110 forming a third support plane 1112 that collectively define a segmented, multi-plane, laterally angled sleep surface 1114 having progressively greater angles of rotation along a longitudinal axis 1115 of support system 1100, from a first or bottom edge 1116 of sleep surface 1114 to an opposing second or top edge 1118 of sleep surface 1114, resulting in relatively greater rotation of the upper respiratory tract of the user (as necessary for efficacy in preventing obstructive apnea) and relatively lesser rotation in the lower body of the user (resulting in greater comfort and perceived stability by avoiding rotation of a majority of the user's body mass). In alternative embodiments, sleep surface 1114 is formed using any suitable number of support pieces defining corresponding support planes, for example, one support piece forming a smooth contour over a length of sleep surface 1114 from first edge 1116 to opposing second edge 1118 or a plurality of support pieces, such as two support pieces, three support pieces, or more than three support pieces forming a smooth contour over the length of sleep surface 1114.

Unlike conventional positional therapies for the prevention of obstructive sleep apnea, which attempt to manipulate the user's sleep position and or orientation using rotation of one plane, in certain embodiments the system described herein uses multiple support planes formed by one or more support pieces to laterally rotate the user. For example, in one embodiment, two support pieces provide two separate support planes, with a first support plane defined by the first support piece configured to support the torso and the legs of the user, and a second support plane defined by the second support piece configured to support the neck and the head of the user.

In an alternative embodiment, three support pieces provide three separate support planes, with a first support plane defined by the first support piece configured to support the legs of the user, a second support plane defined by the second support piece configured to support the torso of the user, and a third support plane defined by the third support piece configured to support the head of the user.

In a further alternative embodiment, more than three support pieces, for example, numerous independent support pieces having a length in a longitudinal direction of sleep surface 1114 of 2-18 inches (50.8-457.2 mm) or, more specifically, 4-12 inches (101.6 - 304.8 mm), or, even more specifically, 6 inches (152.4 mm), provide a corresponding number of separate support planes. Each support piece can be laterally rotated independently of other support pieces to collectively form sleep surface 1114. In a particular embodiment, the numerous support pieces can be combined to form separate support pieces, for example, creating a first support piece having a length of 18 inches (457.2 mm) in the longitudinal direction at the foot of the support system 1100, an adjacent second support piece having a length of 12 inches (304.8 mm) in the longitudinal direction, and a third support piece adjacent the second support piece having a length in the longitudinal direction of 6 inches (152.4 mm). In these embodiments, the support pieces forming the support planes can be rotated as necessary or desired to achieve an optimal configuration that is clinically effective (i.e., prevents apnea) and demonstrates acceptable tolerance (i.e., allows the user to sleep comfortably). In an alternative embodiment, a continuously sloped sleep surface is formed by a plurality of support pieces without step increases in lateral rotational angle; this is illustrated as a sleep surface with an infinite number of support pieces.

In the embodiments described herein, the length in the longitudinal direction of each support piece and defined support plane (and the resulting location of transitions between support planes) is designed to achieve clinical efficacy and tolerability. Therefore, a specific length can be defined in a number of configurations, including without limitations: (a) generic plane dimensions (e.g., based on average body geometry, a length of a torso section of the user defined so that when an average user's head is supported by a head support piece, a transition between the torso support piece and the leg support piece occurs below the user's S3 vertebrae); (b) customized plane dimensions (e.g., a torso support plane has a suitable length in the longitudinal direction appropriate to the user's leg length, torso length, and/or a distance from the user's shoulder to his/her inseam); or (c) dynamic plane dimensions (e.g., transitions selected on dynamic surface appropriate to user, selection being either user-seiected, care-giver defined, or automatically calculated).

In certain embodiments, each support piece defining the corresponding support planes is independently rotatable about an axis extending parallel with a longitudinal axis of the support system. The independent rotation of each support piece allows the caregiver or the user the ability to focus on progressively increasing an angle of rotation in one or more support pieces having support planes positioned to support the torso of the user, and the neck and/or the head of the user. In certain embodiments, an angle of rotation (or lateral rotational angle) at which the one or more support planes defined by the support pieces configured to support the neck and/or the head of the user is positioned is greater than a rotational angle of the one or more support planes defined by the support pieces configured to support the torso of the user, which is greater than a rotational angle at which the one or more support planes defined by the support pieces configured to support the legs of the user is positioned.

In a particular embodiment, the support plane defined by the support piece configured to support the legs and the torso of the user is positioned at a rotational angle of 10° with respect to a base surface of the support piece, while the support plane defined by the support piece configured to support the head of the user is positioned at a rotational angle of 20° with respect to a base surface of the support piece. In an alternative embodiment, a first support plane defined by the support piece configured to support the legs of the user is positioned at a rotational angle of 10° with respect to a base surface of the first support piece, a second support plane defined by a second support piece configured to support the torso of the user is positioned at a rotational angle of 15° with respect to a base surface of the second support piece, and a third support plane defined by the third support piece configured to support the head of the user is positioned at a rotational angle of 20° with respect to a base surface of the third support piece. In alternative embodiments, the support planes can be positioned at any suitable rotational angle including any suitable lateral rotational angle and/or any suitable longitudinal rotational angle.

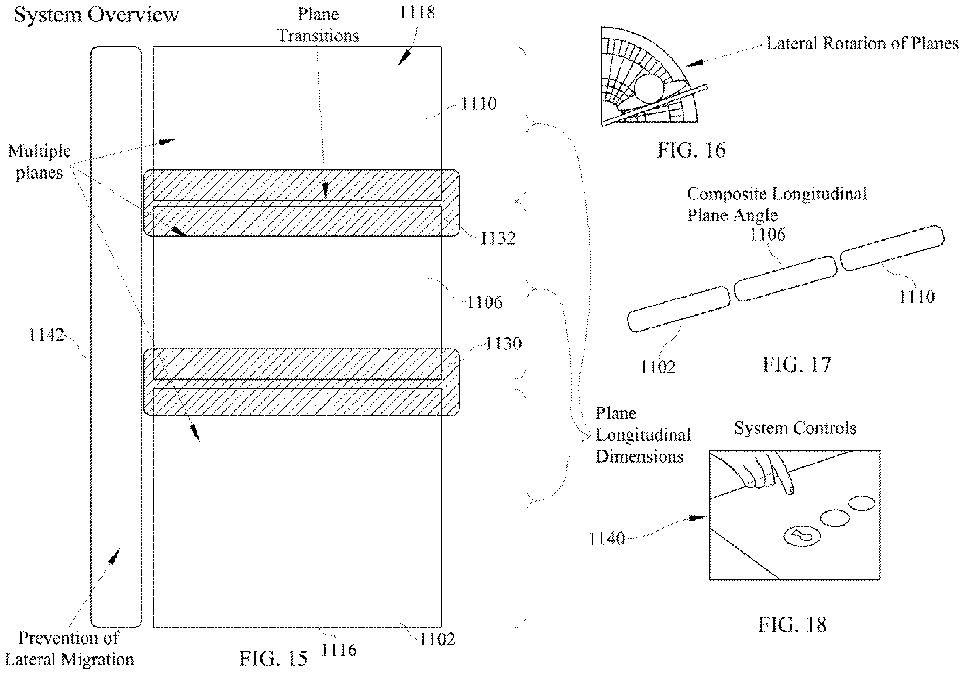

Referring further to FIGS. 14 and 15, in a particular embodiment, first support piece 1102 defines support plane 1104 positioned at a lateral rotational angle α of 20° to 30°, or more specifically, 20° to 25°, or, even more specifically, 25° with respect to a base surface 1122 of first support piece 1102. Second support piece 1106 defines support plane 1108 positioned at a lateral rotational angle β of 10° to 20°, or more specifically, 10° to 15°, or, even more specifically, 15°, with respect to a base surface 1124 of second support piece 1106. Third support piece 1110 defines support plane 1112 positioned at a lateral rotational angle γ of 5° to 15°, or more specifically, 10°, with respect to a base surface 1126 of third support piece 1106. Other lateral rotational angles and step increases in lateral rotational angles between each support piece may also be used to achieve a progressive lateral rotational angle.

In one embodiment as shown in FIG. 15, one or more contoured transitional pieces, such as a first transitional piece 1130 and a second transitional piece 1132, are positionable between adjacent support pieces or at or near a transition line between the adjacent support pieces to provide a gradual continuous transition between support planes. As shown in FIG. 15, in one embodiment, a first transitional piece 1130 is positioned at a transitional line where first support piece 1102 meets with adjacent second support piece 1106 to provide lumbar support for the user. Similarly, a second transitional piece 1132 is positioned at a transitional line where second support piece 1106 meets with adjacent third support piece 1110 to provide lumbar support for the user. In particular embodiments, one or more additional transitional pieces can be positioned on the support planes to provide additional support at the neck region and/or the knee region of the user, for example. In other embodiments, increasing the number of contoured transitional pieces allows for more contouring and gradual changes in the angle of support along the length of the support system 1100.

As shown in FIG. 17, each of first support piece 1102, second support piece 1106, and third support piece 1110 has a respective height in a direction perpendicular to longitudinal axis 1115 of support system 1100. In one embodiment, first support piece 1102 has a maximum height from base surface 1122 to support plane 1116 in a direction perpendicular to longitudinal axis 115 of 14 to 18 inches (355.6 to 457.2 mm), or more specifically, 16 to 17 inches (406.4 to 431.8 mm); second support piece 1106 has a maximum height from base surface 1124 to support plane 1108 in a direction perpendicular to longitudinal axis 1115 of 8 to 12 inches (203.2 to 304.8 mm), or more specifically, 9 to 10 inches (228.6 to 254); and third support piece 1110 has a maximum height from base surface 1126 to support plane 1112 in a direction perpendicular to longitudinal axis 1115 of 4 to 8 inches (101.6 to 203.2 mm), or more specifically, 6 to 7 inches (152.4 to 177.8 mm). As a result, the support pieces can be designed with desired heights and defining support planes desired rotational angles such that support system 1100 provides a composite longitudinal plane angle (e.g., reverse Trendelenburg angle) to facilitate the prevention and/or treatment of sleep apnea as well as to improve tolerability.

As described in greater detail below, in certain embodiments, support system 1100 includes a system controL such as a controller 1140 shown in FIG. 18, having a display configured to display information about support system 1100 including, without limitation, lateral plane angles of each support piece and/or composite plane angles of each support piece. In one embodiment, controller 1140 includes one or more processors configured to adjust the rotational angles of the support planes based on data input by the user or a caregiver and/or data signals received from one or more sensors positioned at locations on or near support system 1100.

Referring again to FIG. 15, in one embodiment, support system 1100 includes a bolster 1142 or other suitable boarder positioned along at least one lateral side of support system 1100 to limit or prevent lateral migration of the user. More specifically, bolster 1142 extends along at least a portion of the lateral side generally parallel with longitudinal axis 1115 to prevent or limit lateral movement of the user positioned on sleep surface 1114 to prevent the user from moving or sliding off sleep surface 1114. Bolster 1142 is bolstered at lower edge 1116 of sleep surface 1114 to define an envelopment zone. In one embodiment, bolster 1142 extends from lower edge 1116 partially along a length of support system 1100 to a torso region of the user, but, in this embodiment, terminates below a head portion of the user. In a particular embodiment, at least a portion of bolster 1142 includes a suitable material to provide a textured surface to facilitate retaining the user in the desired position on the support system 1100. Additionally or alternatively, bolster 1142 may include a formable material, such as a suitable foam material, having one or more different densities along a length of bolster 1142 to provide an increased envelopment throughout sleep surface 1114. A belt and or an adjustable strap (not shown in FIG. 15) or a body may be operatively coupled to bolster 1142 to facilitate maintaining the user properly positioned on sleep surface 1114.

In one embodiment, each of support, pieces 1102, 1106, 1110 are rotatable about Longitudinal axis 1115 to provide sleep surface 1114 having a right side slope or, alternatively, a left side slope to allow the user to sleep on his/her right side or left side, respectively. In one embodiment, one or more cylindrical or tubular sections are positioned within at least a portion of first support piece 1102, second support piece 1106, and third support piece 1110 and coaxially aligned with longitudinal axis 1115 to allow each support piece 1102, 1106, 1110 to rotate about longitudinal axis 1115 independently of the other support pieces 1102, 1106, 1110.

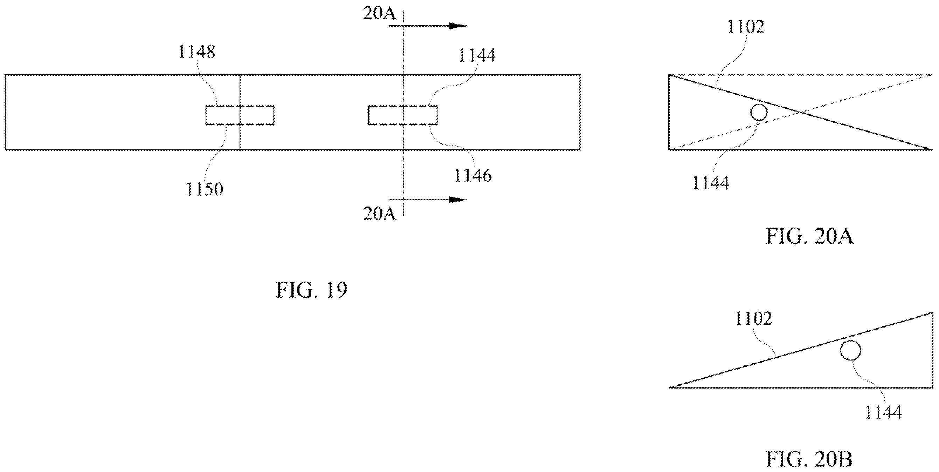

As shown in FIG. 19, a first cylindrical section 1144 is positioned within a bore 1146 defined within a portion of first support piece 1102 and second support piece 1106 along longitudinal axis 1115 to allow first support piece 1102 and second support piece 1106 to rotate about longitudinal axis 1115 and with respect to each other. Similarly, a second cylindrical section 1148 is positioned within a bore 1150 defined within a portion of second support piece 1 106 and third support piece 1110 along longitudinal axis 1115 to allow second support piece 1106 and third support piece 1110 to rotate about longitudinal axis 1115 and with respect to each other. In an alternative embodiment not shown, a single cylindrical section extends through a bore defined through second support piece 1106 and into at least a portion of first support piece 1102 and into at least a portion of third support piece 1110 to allow each of first support piece 1102, second support piece 1106, and third support piece 1110 to rotate about longitudinal axis 1115 and with respect to each other. In this embodiment, each support piece 1102, 1106, 1110 is rotatable between a first orientation having a right side slope, as shown in FIG. 20A, and a second orientation having a left side slope, as shown in FIG. 20B. Axial rotation allows each support piece 1104, 1106, 1110 to lie flat with a right side slope or a left side slope as shown in FIGS. 20A and 20B.

In certain embodiments, support pieces 1102, 1106, 1110 are formed of more than one material, for example, two or more materials, such as two foam materials, having different densities, with the less dense material covering the denser material. In this embodiment, the less dense material is laid on the denser material at the respective base surface and the respective support plane of the support piece to allow sleep surface 1114 to function properly, whether with a right side slope or a left side slope. With the denser material sandwiched between the less dense material, the user will be positioned on the less dense material in either the first or the second orientation.

In this embodiment, support system 1100 allows the user to sleep on either his/her right side or left side, based on the user's sleeping preference. This sleeping preference may not be static. For example, if the user has an injury, an ache, or a desire to change his/her sleeping preference, the orientation of sleep surface 1114 can be changed at any time to accommodate the user's sleeping preference. The orientation can be changed from day to day or during the night. Moreover, from a manufacturing standpoint, a versatile support system 1100 prevents having to manufacture and distribute a sleep surface 1114 having a right side slope and a separate sleep surface 1114 having a left side slope, which would increase production and distribution costs. Finally, a potential purchaser would not have to commit to a sleep side before purchasing the product, which might be a deterrent to purchasing the product.

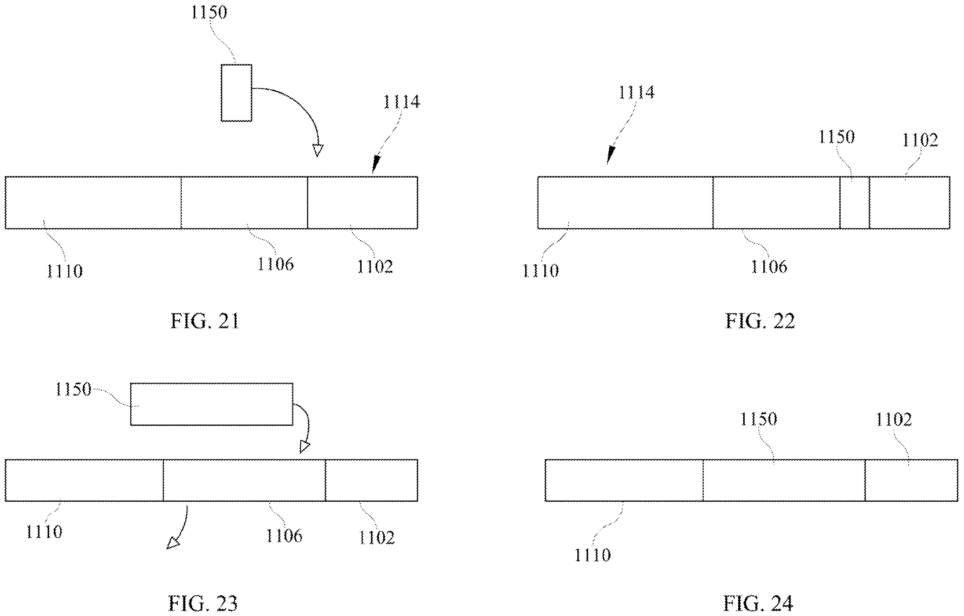

In one embodiment, support system 1100 includes one or more spacers 1150 that allow a length of support system 1100 to be adjusted and customized to a height of the user supported by support system 1100. For example, the length of sleep surface 1114 can be adjusted by adding one or more suitable spacers 1150 or replacing one or more support pieces 1102, 1106, 1110 with a suitable spacer 1150 of a different length, so that transitional lines between lateral angles of support planes defined by adjacent support pieces 1102, 1106, 1110 will desirably occur at a neck region and a hip region of the user. In one embodiment, spacer 1150 has a same or similar lateral rotational angle and/or a same or similar longitudinal rotational angle as the respective lateral rotational angle and the respective longitudinal rotational angle of an adjacent support piece or the support piece that spacer 1150 replaces. In an alternative embodiment, spacer 1150 has a different lateral rotational angle and/or a different longitudinal rotational angle as the respective lateral rotational angle and the respective longitudinal rotational angle of an adjacent support piece or the support piece that spacer 1150 replaces. As shown in FIGS. 21 and 22, spacer 1150 is positioned between first support piece 1102 and second support piece 1106 to adjust the length of sleep surface 1114. As shown in FIGS. 23 and 24, first support piece 1102 is replaced with spacer 1150 to adjust the length of sleep surface 1114.

Assuming the user positions his/her neck at the appropriate location on sleep surface 1114, and an overall length of sleep surface 1114 is adjustable, in one embodiment only second support piece 1106 of support system 1100 has an adjustable length. In alternative embodiments having a support system 1100 with a fixed length, both first support piece 1102 and second support piece 1106 have adjustable lengths. In this embodiment, a length of first support piece 1102 increases as a length of second support piece 1106 decreases and, conversely, the length of first support piece 1102 decreases as the length of second support piece 1106 increases.

In one embodiment, adjacent support pieces 1102, 1106, 1110 and spacers 1150 can be coupled together using a suitable coupling mechanism including, without limitation, one or more of the following: snaps, straps, buttons, and hook-and-loop fasteners. In certain embodiments, the length of sleep surface 1114 is adjustable by any combination of inserting one or more spacers 1150, replacing one or more support pieces 1102, 1106, 1100 with a longer or shorter spacer 1150, cutting or trimming one or more support pieces 1102, 1106, 1110 to a desired length, and removing one or more support pieces 1102, 1106, 1110. In alternative embodiments, the length of sleep surface 1114 is not adjustable but one or more of a leg region, a torso region, and a head region of sleep surface 1114 is adjustable by any combination of inserting one or more spacers 1150, replacing one or more support pieces 1102, 1106, 1100 with a longer or shorter spacer 1150, cutting or trimming one or more support pieces 1102, 1106, 1110 to a desired length, and removing one or more support pieces 1102, 1106, 1110 without adjusting the length of sleep surface 1114.

In a further alternative embodiment, each support piece 1102, 1106, 1110 includes one or more inflatable fluid bladders configured to contain a fluid, such as air. In this embodiment, a length of each support piece 1102, 1106, 1110 is adjustable by adding fluid or removing fluid from one or more respective fluid bladders. By adding fluid to one or more of the respective fluid bladders, the length of the respective support piece 1102, 1106, 1110 is increased and the length of the respective support plane 1104, 1108, 1112 is also increased. Conversely, removing fluid from one or more of the respective fluid bladders, the length of the respective support piece 1102, 1106, 1110 is decreased and the length of the respective support plane 1104, 1108, 1112 is also decreased. The amount of fluid within the respective fluid bladders can be monitored and controlled electronically or by the user or caregiver using a suitable device including, without limitation, a suitable pneumatic pump or nozzle. In certain embodiments, a coupler, such as one or more snaps or straps, are utilized to maintain the desired amount of fluid within the respective fluid bladders and provide additional support to the respective support plane(s), for example, when the fluid bladders are not inflated.

As described herein, sleep surface 1114 is customizable to anthropometric dimensions of the individual user to facilitate support system 1100 performance that optimizes or matches the design intent - the body position of the user will prevent or limit undesirable sleep apnea episodes and provide improved comfort.

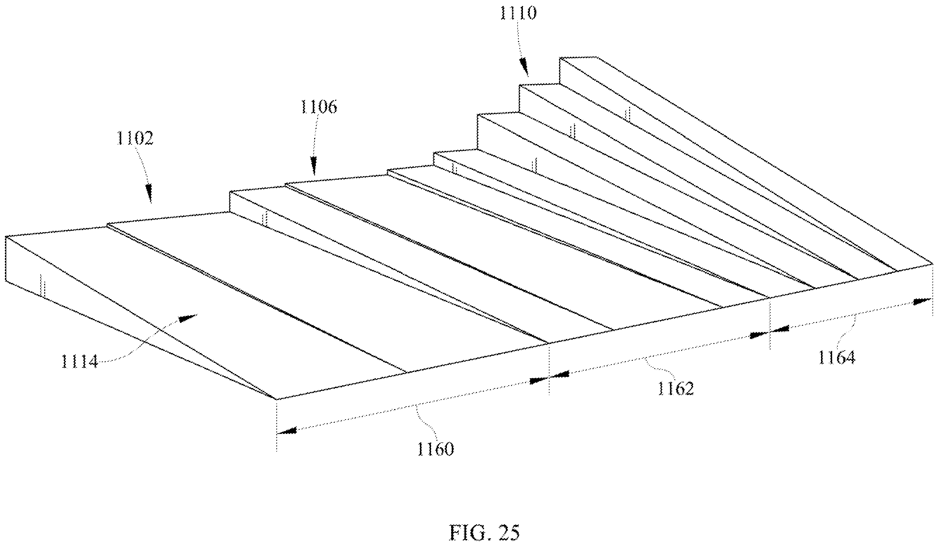

In certain embodiments, support system 1100 includes a plurality of support pieces, such as two support pieces, three support pieces, or more than 3 support pieces, and more specifically, at least 6 support pieces, and even more specifically, 8-20 support pieces. For example, referring to FIG. 25, in one embodiment each of a leg region 1160 corresponding to first support piece 1102, a torso region 1162 corresponding to second support piece 1106, and a head region 1164 corresponding to third support piece 1110 of support system 1100 includes a plurality of independent support wedges forming a finer gradation in the longitudinal slope of sleep surface 1114 to increase user compliance and the effectiveness of support system 1100 in preventing or limiting sleep apnea episodes and providing more comfort for the user supported on sleep surface 1114. The support wedges may be formed of one or more suitable materials including, without limitation, a formable material, a semi-rigid material, a foam material or one or more fluid bladders.

In the embodiment shown in FIG. 25, first support piece 1102 includes two independent support wedges defining respective support planes positioned at different lateral rotational angles, second support piece 1106 includes three independent support wedges defining respective support planes positioned at different lateral rotational angles, and third support piece 1110 includes four independent support wedges defining respective support planes positioned at different lateral rotational angles. In alternative embodiments, each support piece 1102, 1106, 1110 includes any suitable number of independent support wedges. Generally, an increasing number of independent support wedges within a selected support piece allows for more detailed and specific contouring of sleep surface 1114 and more gradual changes in rotational angles of adjacent support wedges and support pieces along the length of sleep surface 1114.

For example, a support system including a series of support wedges may twist or urge the user's body to rotate and tilt the user's head in a more gradual trend than a support system including only three larger support pieces with respective support planes of different lateral rotational angles. The additional support wedges allow for more comfortable transitions between and within the lower body, the torso, and the upper body of the patient. The increased number of support wedges allow for more specific positioning of the patient's body, and a more effective therapy.

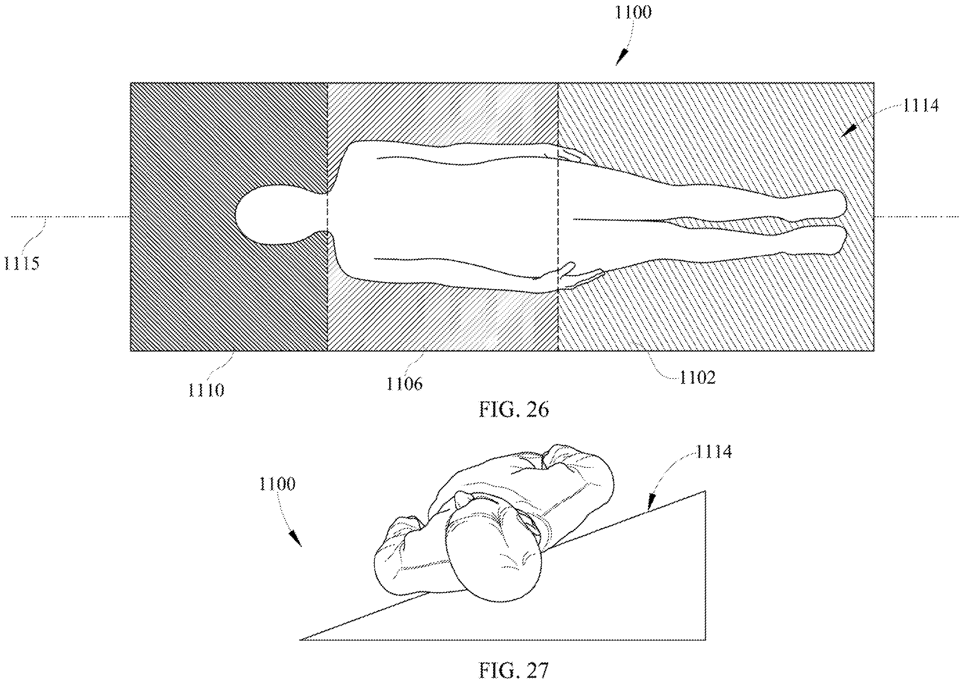

Referring to FIGS. 26 and 27, in one embodiment, each support piece 1102, 1106, 1110 defines a support plane positioned at the same or similar lateral rotational angle; however, each support piece 1102, 1106, 1110 is made of a material having a different density than the material used to make the other support pieces. The base material of each support piece 1102, 1106, 1110 may be the same or different than the base material of the other support pieces, but with a different density. In a particular embodiment, support system 1 100 utilizes varied foam density to achieve a variation in the lateral rotation of the user's body across different body segments. In one particular embodiment, support piece 1102 is composed of the least dense material, support piece 1106 is composed of the medium density material, and support piece 1110 is composed of the most dense material.

In this embodiment, sleep surface 1114 is formed of support pieces cut to form support planes at the same lateral rotational angle but with different densities. To achieve a greater relative rotation at the head portion of the user, third support piece 1110 is denser than second support piece 1106, while first support piece is less dense than second support piece 1106 and third support piece 1110 to achieve a lesser or limited relative rotation at the leg region of the user. In a particular embodiment, rather than having a plurality of discrete support pieces, sleep surface 1114 is one continuous support piece exhibiting a gradual density transition along a longitudinal length of sleep surface 1114 such that the leg portion of sleep surface 1114 is less dense than the opposite head portion of the sleep surface 1114. This feature may result in a support system that appears less intimidating to the user and more aesthetically pleasing. Moreover, sleep surface 1114 is rotatable about longitudinal axis 1115, shown in FIG. 26, so that sleep surface 1114 is oriented in one of a lateral right side slope or a lateral left side slope shown in FIG. 27.

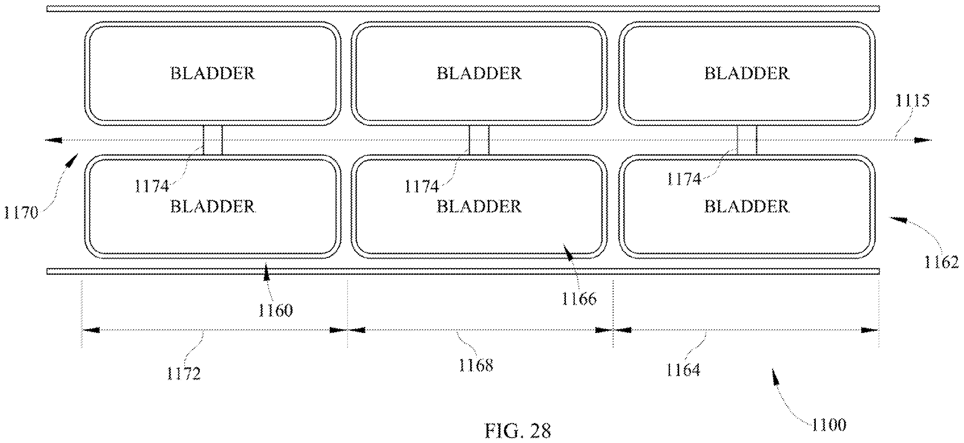

Referring to FIGS. 28-35, in an alternative embodiment, sleep surface 1114 is formed of a closed air system 1160 that induces the user's body to rotate laterally when sleeping to facilitate preventing or limiting the incidence of sleep apnea. In certain embodiments, closed air system 1160 does not require electrical power or control, and allows the user to quietly move sleep orientations between the lateral left side slope and the lateral right side slope during sleep.

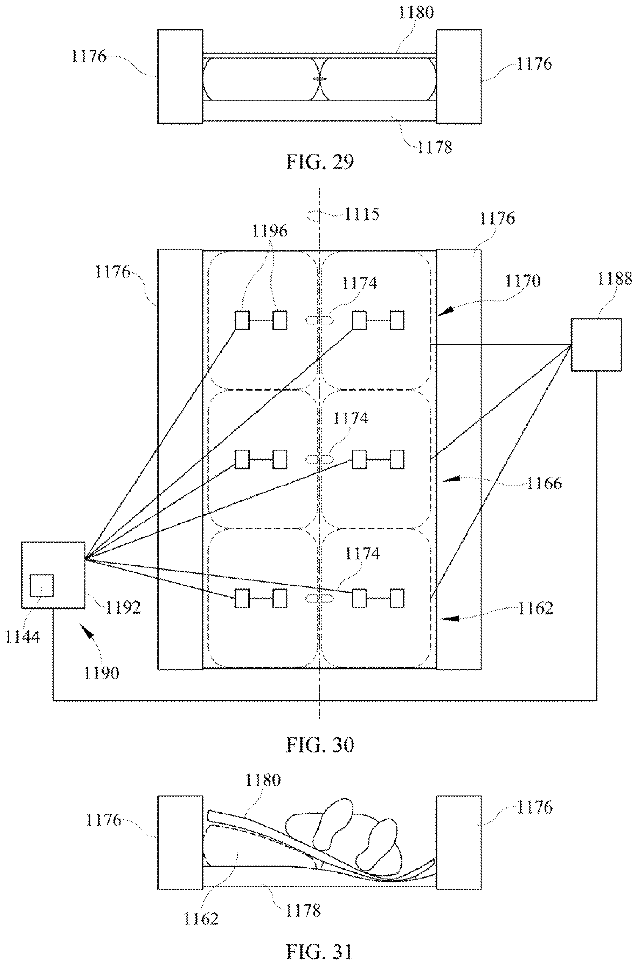

In one embodiment, closed air system 1160 includes one or more pairs of fluid bladders communicatively coupled to each other. For example, as shown in FIGS. 28 and 30, a first pair of fluid bladders 1162 is positioned within the leg region 1164 of closed air system 1160, a second pair of fluid bladders 1166 is positioned within a torso region 1168 of closed air system 1160, and a third pair of fluid bladders 1170 is positioned within a head region 1172 of closed air system 1160. In a particular embodiment, a sleep sensor is positioned in a pillow or on the bladder 1166. In a particular embodiment, the fluid bladders are plumbed together using a suitably sized tube or hose, shown schematically by reference number 1174 in FIGS. 28 and 30, or any suitable coupling mechanism providing communication between the interior cavities of the fluid bladders to allow fluid to move at a desired rate between the coupled bladders. Fluid, such as air, can be added manually or using a suitable pump to each pair of fluid bladders, for example, through one or more nozzles to adjust the firmness and lateral rotational angle of the respective pair of fluid bladders. In this embodiment, the user can adjust the side upon which he/she sleeps (even during sleep) and an amount of fluid contained within the fluid bladders to adjust the firmness of sleep surface 1114 and/or the lateral rotational angle of each support plane forming sleep surface 1114. In this embodiment, each pair of fluid bladders is separated along longitudinal axis 1115 of support system 1100. In a particular embodiment, fluid can be added to the bladders 1166 based on the sleep state of the person.

In a particular embodiment, closed air system 1160 includes one or more bolsters 1176, as shown in FIGS. 29-31, positioned along at least a portion of the opposing lateral sides of support system 1100 to prevent or limit lateral migration of the user during sleep. In one embodiment, bolsters 1176 are the same or similar to bolster 1142 described above with reference to FIG. 15. Referring further to FIG. 29, each air bladder rests on and is supported by a suitable bottom layer, such as a foam material layer 1178 and/or a mattress, and can also be covered by another suitable top layer, such as a foam material layer 1180. Materials other than foam materials known to those having ordinary skill in the art can be utilize to form the bottom layer and/or the top layer. In a certain embodiment, material layer 1180 at least partially encloses or envelops one or more of the fluid bladders to retain the fluid bladders properly positioned within support system 1100. One or more of the fluid bladders in one or more of the pairs of fluid bladders are inflatable to rotate the user onto his/her right side or left side based at least in part on his/her sleep state.

In one embodiment, one or more pairs of fluid bladders 1162, 1166, 1170 include two wedge-shaped fluid bladders that are removably coupled to material layer 1178 and/or material layer 1180 using a suitable coupler, such as a hook and loop fastener system. For example, third pair of fluid bladders 1170 are positioned with respect to the user's upper body or head region and are removably coupled to material layer 1180 using a hook and loop fastener system such that sleep surface 1114 is adjustable based at least in part on the size and weight of the user. These fluid bladders are inflatable based on the user's sleep state to urge the upper body of the user to rotate. Additionally, first pair of fluid bladders 1162 and/or second pair of fluid bladders 1166 are also inflatable to urge the user's legs and/or the user's torso, respectively, to rotate.

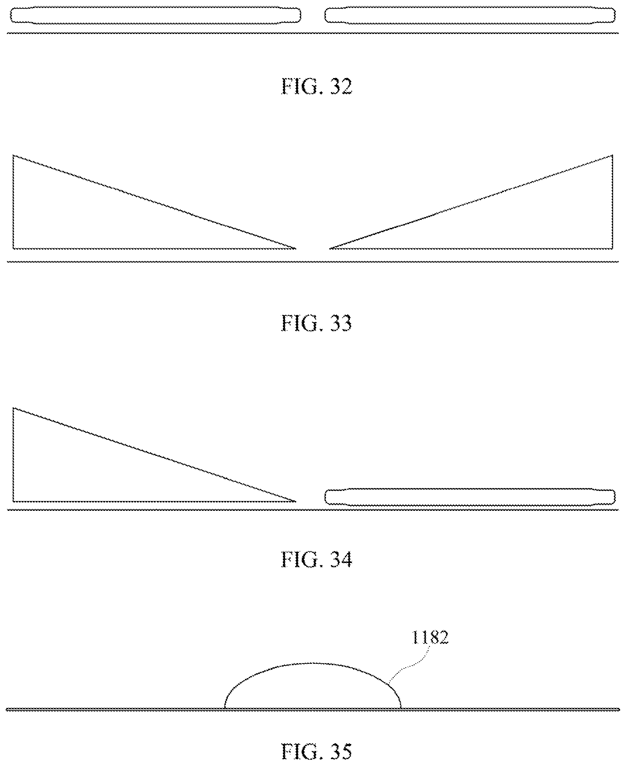

Referring further to FIGS. 32-35, each fluid bladder of each pair of fluid bladders 1162, 1166, 1170 is inflatable to form a support piece having a desired or selected shape. Select fluid bladders may remain substantially deflated, as shown in FIG. 32, or both fluid bladders or only one fluid bladder of one or more pairs of fluid bladders may be inflated to form a desired sleep surface 1114, as shown in FIGS. 33 and 34 respectively. In an alternative embodiment, as shown in FIG. 35 a single fluid bladder 1182 may be utilized in one or more of leg region 1164, torso region 1168, and head region 1172 of closed air system 1160 positioned along longitudinal axis 1115 of support system 1100 that, when inflated, urges the user to roll towards either the lateral right side or the lateral left side after the user is in a predetermined sleep state. In this embodiment, fluid bladder 1182 can be deflated occasionally to allow the user to reposition himself/herself. A pillow can be positioned on third pair of fluid bladders, for example, such that the pillow is inclined when one or more of the fluid bladders are inflated.

The fluid bladders are inflatable with air or another suitable fluid (which can be drained as desired from within the cavities of the fluid bladders into a reservoir). A fluid supply 1188, shown in FIG. 30, is positioned at or near support system 1100, such as on the floor, beneath the bed, or coupled to the bed. The fluid supply is in independent fluid communication with each pair of fluid bladders 1162, 1166, 1170 to supply a desired amount of fluid to each fluid bladder based on a signal from a control, for example.

In one embodiment as shown in FIG. 30, support system 1100 includes a suitable computer-implemented control system 1190 operatively coupled to closed air system 1160, such as in operational control communication with closed air system 1160. The computer-implemented control system includes a computer 1192 having one or more processors 1194 and one or more sleep sensors 1196, such as one or more pressure sensors, coupled in signal communication with processors 1194. Sleep sensors 1196 are configured to monitor the user's sleep patterns and transmit signals indicative of the sensed sleep patterns to processors 1194 for manipulation and evaluation of the data. Based at least in part on the one or more signals received from one or more sleep sensors 1196, control system 1190 is configured to inflate or deflate select fluid bladders to reposition the user dining sleep to prevent or limit the occurrence of a sleep apnea episode, for example.

Additionally, in certain embodiments, closed air system 1160 is configured to rest on a conventional mattress or may be configured or reinforced to rest directly on a support structure, such as a bed frame or a floor. With the fluid substantially removed from each of the fluid bladders, closed air system 1160 can be folded or rolled into a compact configuration to facilitate storing and transporting closed air system 1160. In certain embodiments, closed air system is less expensive than a conventional mattress and more compact to facilitate portability of support system 1100. Additionally, closed air system 1160 as configured prevents or limits disturbance to the user's partner sleeping next to the user.

In certain embodiments as described herein, support system 1100 is a dynamic support system, rather than a static support system, that is configured to control the configuration of sleep surface 1114 based at least in part on data entered into control system 1190 using computer 1192, or another control operatively coupled to computer 1192, and/or sensed by one or more sleep sensors 1196, for example, to improve the performance of sleep surface 1114 in terms of clinical efficacy and user tolerability.

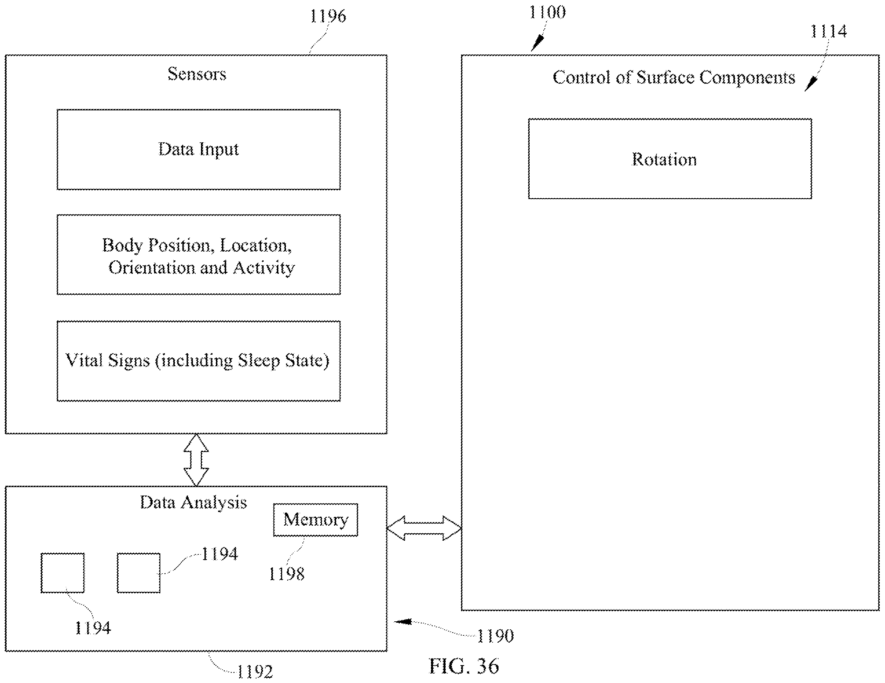

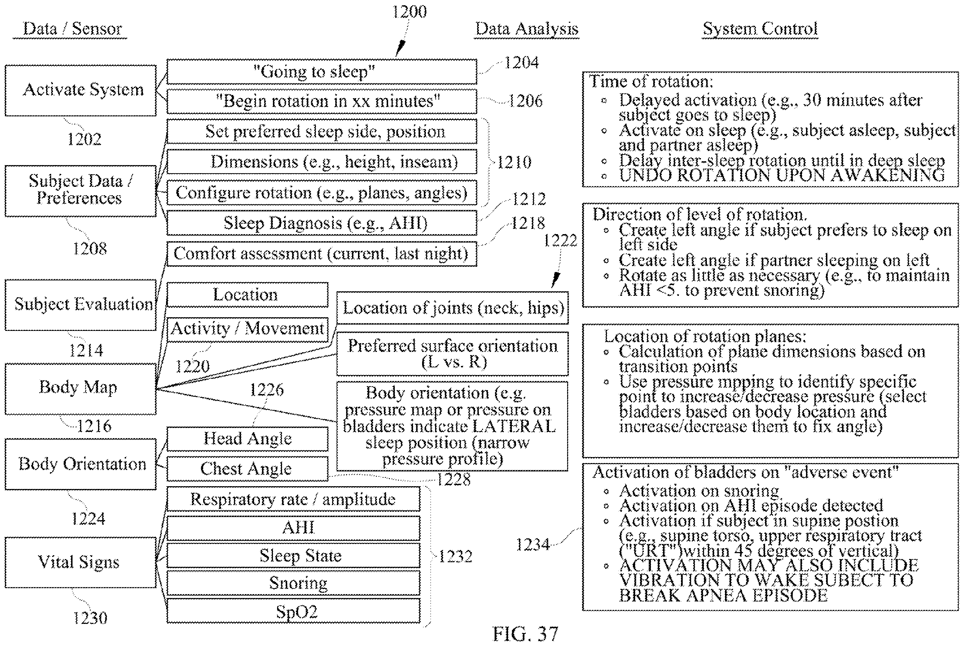

As described herein and shown schematically, for example, in FIGS. 36 and 37, dynamic support system 1100 includes, in addition to other components, a plurality of sleep sensors 1196 configured to sense and monitor various activities including without limitation, the user's body position, a location of the user with respect to sleep surface 1114, an orientation, for example, a left side orientation or a rights side sleep orientation, of the user, the user's vital signs including his/her sleep state, and additional relevant user activity during sleep. Each sleep sensor 1196 is in signal communication with one or more processors 1194 contained within computer 1192 and configured to gather relevant data and generate and transmit to processors 1194 signals indicative of the data gathered. Sleep sensors 1196 are also configured to receive operation control signals from processors 1194.

Within computer 1192, data received from sleep sensors 1196 is analyzed and operational control signals are transmitted to sleep sensors 1196 as well as to other components of support system 1100, such as to fluid supply 1188 to activate fluid supply 1188 to provide air to one or more fluid bladders and/or remove air from one or more fluid bladders to adjust steep surface 1114 based on signals generated by sleep sensors 1196 and analyzed within computer 1192. In one embodiment, computer 1192 includes suitable memory 1198 to store data sensed and/or generated by control system 1190.

An exemplary method 1200 utilizing control system 1190 for monitoring the sleep activities of a user positioned on support system 1100 is illustrated in FIG. 37. As described above, control system 1190 includes one or more processors 1194 configured to perform the steps as described herein.

Control system 1190 is activated 1202 either manually or automatically to monitor the user's sleep activities and patterns as user begins to sleep. In one embodiment, control system 1190 detects when the user begins to fall asleep 1204 and activates support system 1100 (or a dynamic sleep surface) on a delay 1206 to rotate the user at a suitable time after sleep is detected, such as after the user has been asleep for 30 minutes. In an alternative embodiment, control system 1190 is programmed to activate support system 1100 at a preset time, for example, at a 30 minute delay, without relying on monitoring the user's sleep activity. In a particular embodiment, control system 11 90 delays inter-sleep rotation of the user until the user is in a deep sleep. Further, when control system 1190 detects that the user is waking, control system 1190 will activate support system 1100 to move sleep surface 1114 to an initial configuration such that the user can exit from support system 1100. In a further embodiment, control system 1190 prevents activation of support system 1 100 if control system 1190 detects the user is sleeping in a lateral decubitus position.

Prior to sleep, the user is able to input 1208 to control system 1190 sleep data 1210 including without limitation, preferred sleeping sides and positions, the user's measurements including, for example, the user's height, weight, and inseam and torso measurements, preferred lateral rotational angles and/or longitudinal rotational angles of one or more support planes defining sleep surface 1114. Based at least in part on the user's input data, control system 1190 is configured to activate support system 1100 to adjust a direction and/or a level of rotation of one or more support planes defining sleep surface 1114. For example, if the user prefers a left side slope to sleep surface 1144, control system 1114 activates fluid bladders within support system 1100 to form the desired lateral left side slope, or if the user's partner is sleeping on the left side of the user, a left angle may be created. In one embodiment, minimal adjustments are made to sleep surface 1114 to maintain the user's AHI under 5 and/or prevent snoring because apneas events and snoring may or may not be equivalent, depending on the user. Additionally, control system 1190 is configured to collected and record data obtained as the user sleeps to diagnose any undesirable or abnormal sleep activities or conditions, including the user's apnea-hypopnea index (AHI), for example.

During sleep, control system 1190 assesses the user's comfort level 1214 and, in a particular embodiment, compares the current evaluation with previous evaluations. The user's body is then mapped 1216 to map body region locations 1218, and user activities and movements 1220 during sleep. The collected data is then analyzed 1222 to determine: the location of joints including, for example, the user's neck, hips, and knees; preferred surface orientation (right side vs. left side orientation); and body orientation (e.g., mapping pressures at various locations on sleep surface 1114 as a result of the user's body orientation, for example, a lateral sleep position indicated by a narrow pressure mapping profile). In one embodiment, location of one or more support planes are calculated and located based on transition points. Under the pressure mapping, specific pressure points are identified to increase or decrease pressure. For example, select fluid bladders are inflated or deflated based on body location and desired lateral rotational angles.

Control system 1190 then assesses 1224 the user's body orientation including, for example a determination of head angle 1226 and chest angle 1228. During sleep, control systems also actively monitors 1230 the user's vital signs, which includes measuring and monitoring the user's respiratory rate and amplitude, AHI, sleep state, snoring, and oxygen saturation (SpO₂), for example. If an adverse event is detected, control system 1190 activates 1234 one or more components of support system 1100 to respond appropriately. For example, fluid supply 1188 may be activated to inflate or deflate one or more fluid bladders. Control system 1190 may activate fluid supply 1188 based on one or more of the following events: detection of snoring, detection of an AHI episode (apnea and/or hypopnea), and detection that the user is in a supine position (e.g., supine torso, upper respiratory tract (URT) within 45° of vertical). Control system 1190 may also activate support system 1100 to vibrate to wake the user should control system 1190 detect an adverse event, such as an apnea episode.

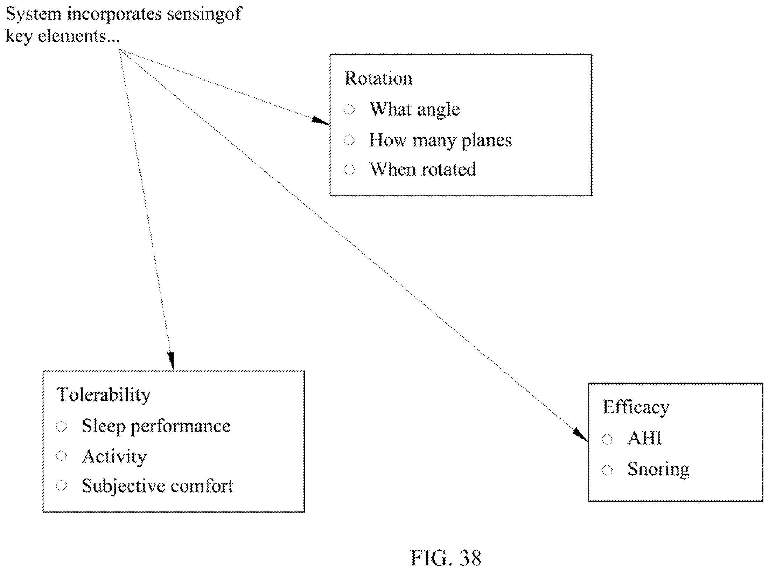

Referring to FIGS. 38-41, in one embodiment a sleep apnea therapy system is a design based on how an average user responds to the therapy tested on a sufficiently large population. The sleep apnea therapy system will effectively and tolerably treat any user's sleep apnea. As a result, the therapy can be modified to decrease a level of therapy (specifically, an amount of rotation) and still achieve clinical efficacy while optimizing user comfort and increasing usage compliance. By learning how the user reacts to variations in therapy, the sleep apnea therapy system is adjustable to optimize the results of therapy. As shown in FIGS. 38-41, the sleep apnea therapy system is design to include, in the embodiment illustrated, an active control of surface (e.g., rotation planes, rotation angles, rotation time/duration, fluid bladder pressure); capabilities to sense and assess tolerability (e.g., sleep state/stage, vitals, movement, user assessment); and sense and assess clinical efficacy (e.g., AHI, respiratory rate, head orientation). With these assessments, tolerability and user compliance is maximized for a clinically effective treatment of obstructive sleep apnea.

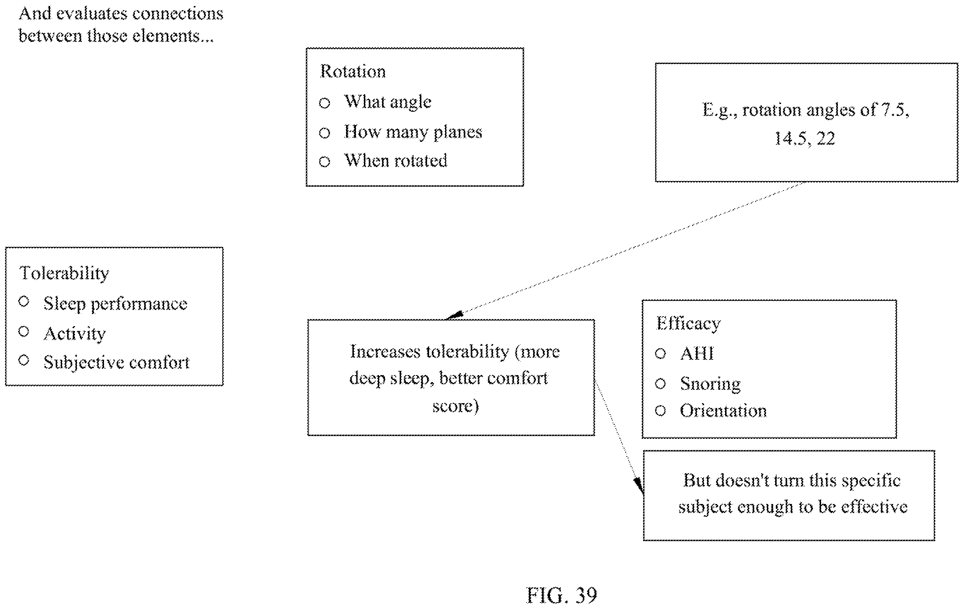

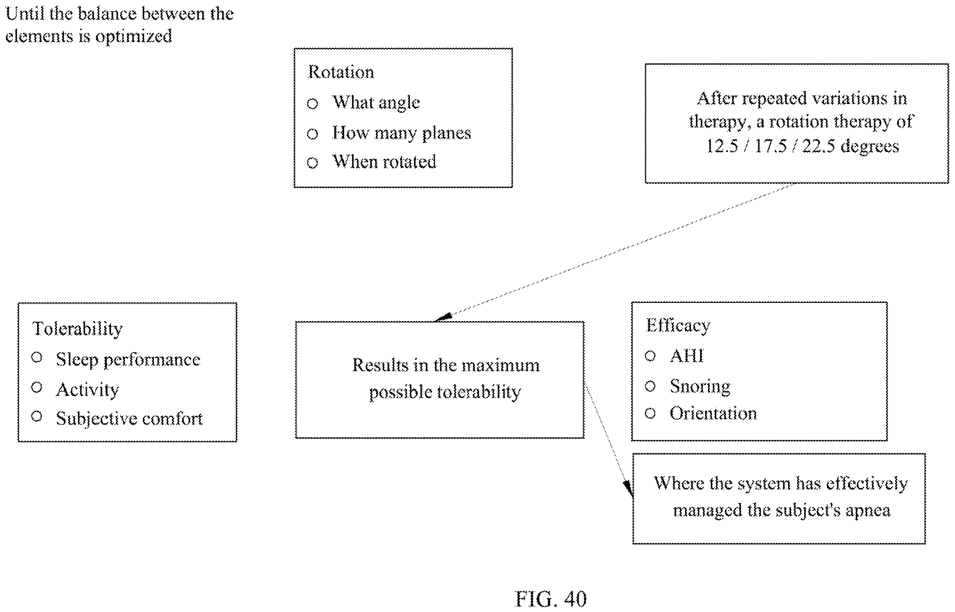

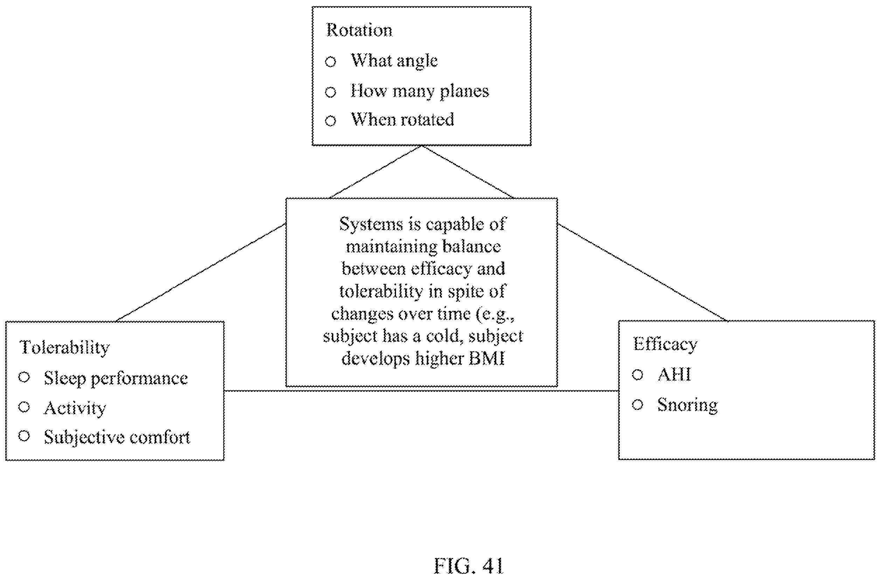

As shown in FIG. 38, the sleep apnea therapy system incorporates sensing of key elements and evaluates connections between those elements until the balance between the elements is optimized, as shown in FIGS. 39 and 40. As a result, the system is capable of maintaining balance between efficacy and tolerability in spite of changes over time (e.g., the user has a cold or develops a higher body mass index (BMI) illustrated in FIG. 41.

In one embodiment, apnea therapy can be integrated as an option in a continuous lateral rotation therapy (CLRT) system. An exemplary CLRT system is configured to deliver lateral rotation as a therapy for the prevention of pressure ulcers, as well as for use in the prevention of ventilator-associated pneumonia and muscular wasting associated with prolonged immobility. The exemplary CLRT system is suitable for use as a therapy for the prevention of sleep apnea with the addition of the following components or elements. In one embodiment, the CLRT system includes a restrained lateral rotation to create or develop progressively greater rotation by limiting rotation in a torso region and/or a head region of the user. Additionally, an augmented lateral rotation increases rotation in the torso region and/or the head region of the user.

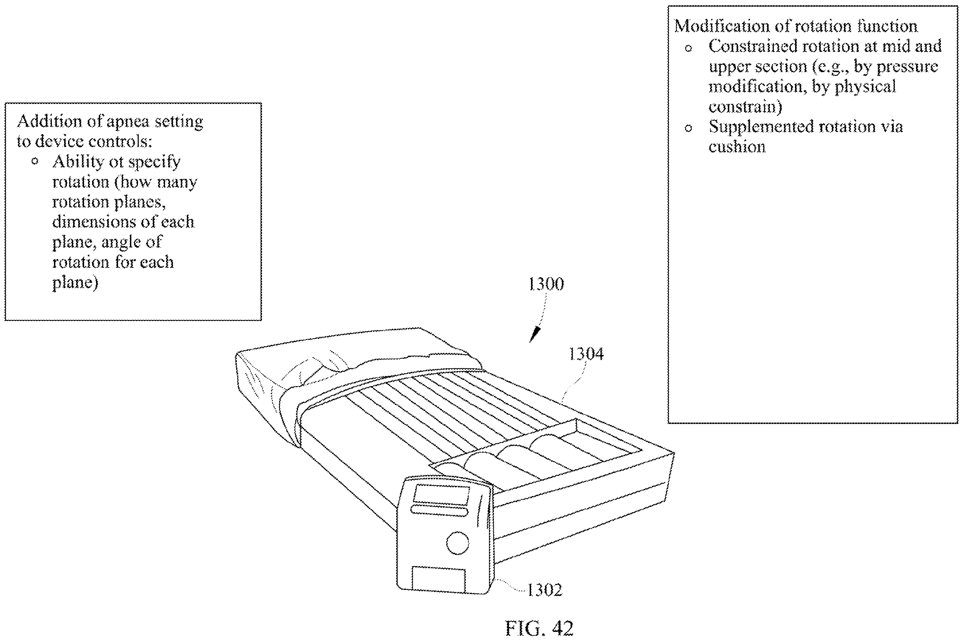

Referring to FIGS. 42-46, an exemplary CLRT system 1300 includes a control system 1302 configured with a rotation function (augmented or restrained rotation). Referring to FIG. 42, control system 1302 is operatively coupled to a support system 1304. Control system 1302 includes an apnea setting configured to select a number of support planes, dimensions of each support plane, and a desired lateral rotational angle and/or a desired longitudinal rotational angle at which one or more support planes are positioned to define the sleep surface. Further, control system 302 includes a rotation function that allows constrained rotation at a torso region and/or a head region of the sleep surface (e.g., by pressure modification or by physical constraint), as well as supplemented rotation via a cushion.

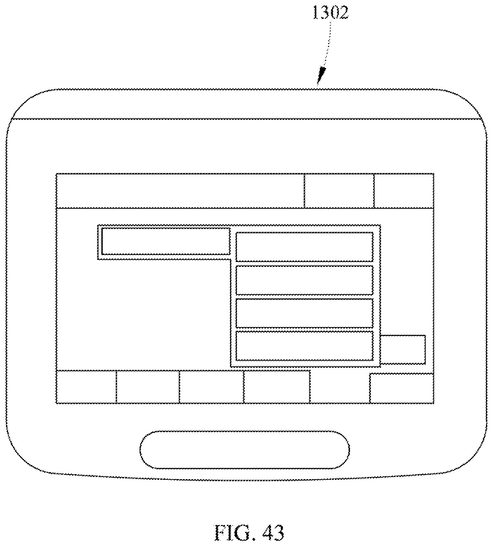

As show in FIG. 43, control system 1302 includes an apnea mode, wherein blowers are controlled to initiate and maintain rotation of the support planes. Within the apnea mode, control system 1302 allows the user or a caregiver to select and/or define one or more therapy modes (e.g., an amount and/or a location of rotation). In one embodiment, control system 1302 is configured or programmed to suggest rotation protocol based on sensed or input data including, without limitation, one or more of the following: AHI score, BMI, sensed respiratory rate, and sensed SpO₂ history. Control system 1302 is also configured or programmed to select a left side slope or a right side slope based on user preference or an alternating lateral rotation, select a reverse trend or composite longitudinal angle, and manually cancel a protocol and/or return the sleep surface immediately to a flat, initial position. Alternating lateral rotation can be specified to alternate after an elapsed time period, to rotate at a certain speed to avoid waking the user, and to gradually increase lateral rotational angles from a low initial lateral rotational angle at a first rotation toward the maximum desired lateral rotational angle after a specified number of rotations.

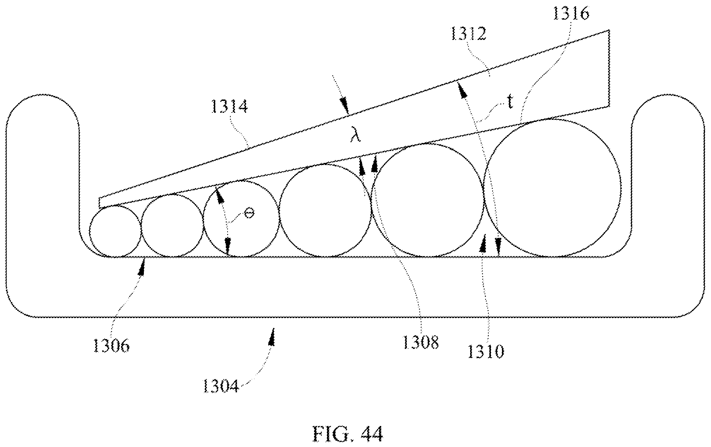

In one embodiment, support system 1304 includes a base support 1306 including a plurality of inflatable fluid bladders aligned generally parallel to a longitudinal axis of support system 1304 forming a single support plane 1308 having a lateral rotation angle θ of 5° to 15°, or more specifically, 10°, with respect to a base plane 1310 of base support 1306. One or more supplemental support wedges 1312 are positioned on support plane 1308 within one or more of the leg region, the torso region, and the head region of support system 1304. In this embodiment, supplemental support wedge 1312 is a wedge-shaped inflatable fluid bladder. As shown in FIG. 44, supplemental support wedge 1312 is positioned at the head region of support system 1304 and forms a supplemental support plane 1314 having a lateral rotation angle λ of 5° to 15°, or more specifically, 10°, with respect to a base plane 1316 of supplemental support wedge 1312. As a result, in the embodiment shown in FIG. 44 a supplemental support plane 1314 is positioned at a total lateral rotational angle *t* of 20° with respect to base plane 1310 of base support 1306 (the sum of angle θ and angle λ). In alternative embodiments, the total lateral rotational angle may be any suitable angle, less than 20° or greater than 20°. Further, one or more supplemental support wedges 1312 can be positioned within one or more of the leg region, the torso region, and the head region of support system 1304.

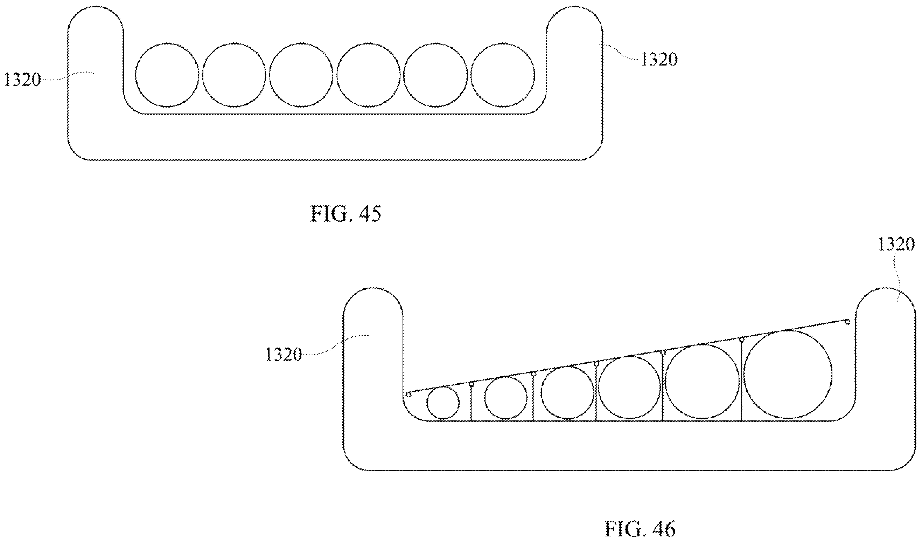

Referring to FIGS. 45 and 46, in one embodiment support system 1304 includes laterally positioned side constraints 1320 to limit inflation of individual fluid bladders forming base support 1306. Support system 1304 may include, with or without laterally positioned side constraints 1320, a plurality of fixed length bands 1322 positioned with respect to individual fluid bladders, such as between adjacent fluid bladders, to limit inflation of the individual fluid bladders.

In one embodiment, a posture garment or shirt 1500 is worn by a user suffering from sleep apnea to apply an appropriate force, such as a tugging force, on the shoulders, arms, and/or head of the user to urge or cause the desired or necessary head turn to open up the user's upper respiratory tract to prevent or limit the occurrence of sleep apnea or in the event of a sleep apnea episode. Applying forces to cause the user to turn his/her entire body to the lateral decubitus position is an alternative approach to achieving this desired head angle; this may involve the use of whole-body garments or a pant garment in combination with a shirt garment.

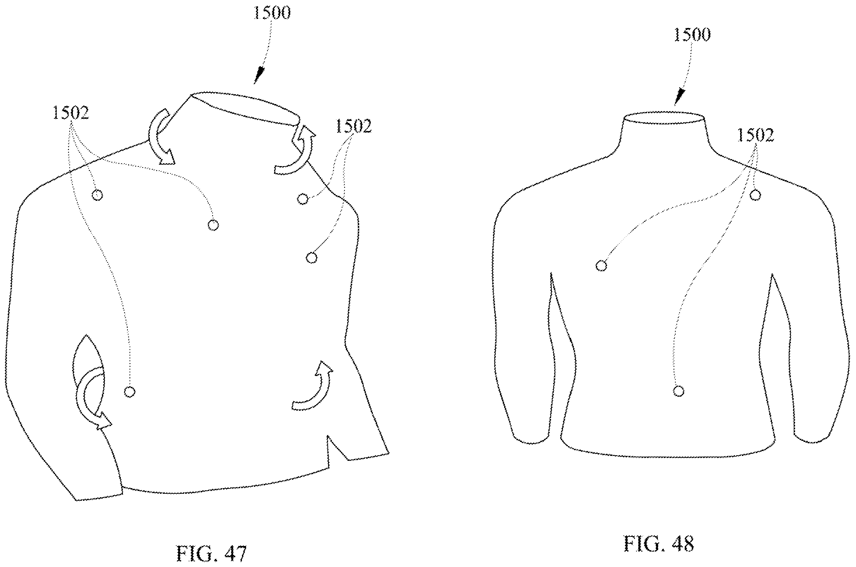

As shown in FIGS. 47 and 48, posture shirt 1 500 includes one or more areas 1502 located on a front portion of posture shirt 1500, as shown in FIG. 47, and/or a back portion of posture shirt 1500, as shown in FIG. 48, including a material panel and/or material weaves having a different elasticity than other areas of posture shirt 1500. Because of the different material elasticity within areas 1502, areas 1502 tend to pull or urge select parts of the user's torso, extremities, head, and/or neck in a desired direction to open the upper respiratory airway. In a particular embodiment, sections or panels of posture shirt 1500 within areas 1502 are made of a different elastic material that work cooperatively to properly position the user's body. Posture shirt 1500 may have long sleeves, short sleeves, or may not include sleeves, and/or have a hood. Any suitable material known to those having ordinary skill in the art may be used within areas 1502 and include, without limitation, elastic materials based on composition (one or more of nylon, polyester, polyester fleece, and/or cotton) or weave (one or more of plain, basket, and twill weaves) that impart preferential deformability and recovery inducing a change in the user's posture.

In one embodiment, a compression posture shirt 1500 is worn like a typical shirt and naturally twists the torso, neck and/or head of the user. Unlike conventional posture shirts, there is no need to insert bladders or tennis ball-like inserts to urge the user to turn or rotate from a supine sleep position. Moreover, compression posture shirt 1500 for sleep apnea does not require any user training because posture shirt 1500 pulls and tugs on the user without the need of intervention from the user.

In certain embodiments, electrical circuitry, such as one or more processors and/or one or more circuit boards, is operatively coupled to, such as in electrical or electronic communication with, control system 1190 to monitor operation of one or more components of support system 1100 or control system 1302 to monitor operation of one or more components of support system 1304, collect, process, and/or store information, such as operation data and motor usage data, and transmit information, such as operation data and motor usage data, to one or more of the following computer-implemented machines or devices including, without limitation, a control and/or display device within or operatively coupled to support system 1100 or support system 1304, and/or a control and/or display device on a computer or network of computers at one or more nurse stations or administrative stations, for example.

In one embodiment, electrical circuitry, such as one or more processors and/or one or more circuit boards, is contained within control system 1190 or control system 1302 and connected in communication with support system 1100 or support system 1304, respectively. In a particular embodiment, one or more sensors or other suitable detection components are operatively coupled to support system 1100 or support system 1304 and/or control system 1190 or control system 1302 to detect operation. The one or more sensors are configured to generate and transmit electronic signals representative of the detected operation to the circuit board, which is configured to collect, process, and/or store such information, and generate and transmit information to one or more computer-implemented machines or devices in communication with the circuit board, as described above.

In certain embodiments, the one or more computer-implemented machines or devices in communication with the circuit board include a controller in signal communication, either wired or wireless signal communication, with the circuit board contained within support system 1100 or support system 1304. The controller includes a suitable display to display information received from the circuit board and/or information generated by the controller based on the information received from the circuit board. In a particular embodiment, the controller is configured to generate command signals and transmit the command signals to the circuit board contained within support system 1 100 or support system 1304 to control operation of support system 1100 or support system 1304 and/or adjust parameters and/or limits, for example, programmed into the circuit board.

The above embodiments may be described in terms of functional block components and various processing steps. Such functional blocks may be realized by any number of hardware and/or software components configured to perform the specified functions. For example, embodiments may employ various integrated circuit components, e.g., memory elements, processing elements, logic elements, look-up tables, and the like, which may carry out a variety of functions under the control of one or more processors, microprocessors or other control devices. Similarly, where the elements of the above embodiments are implemented using software programming or software elements the embodiments may be implemented with any programming or scripting language such as C, C++, Java, assembler, or the like, with the various algorithms being implemented with any combination of data structures, objects, processes, routines or other programming elements. Furthermore, the embodiments could employ any number of conventional techniques for electronics configuration, signal processing and/or control, data processing and the like. The word mechanism may be used broadly and is not limited to mechanical or physical embodiments, but can include software routines in conjunction with processors, etc.

The particular implementations shown and described herein are illustrative examples of the invention and are not intended to otherwise limit the scope of the invention in any way. For the sake of brevity, conventional electronics, control systems, software development and other functional aspects of the systems (and components of the individual operating components of the systems) may not be described in detail. Furthermore, the connecting lines, or connectors shown in the various figures presented are intended to represent exemplary functional relationships and/or physical or logical couplings between the various elements. It should be noted that many alternative or additional functional relationships, physical connections or logical connections may be present in a practical device. Moreover, no item or component is essential to the practice of the invention unless the element is specifically described as "essential" or "critical." Numerous modifications and adaptations will be readily apparent to those skilled in this art without departing from the spirit and scope of the embodiments.

The order of execution or performance of the operations in embodiments illustrated and described herein is not essential, unless otherwise specified. That is, the operations may be performed in any order, unless otherwise specified, and embodiments as described may include additional or fewer operations than those disclosed herein. For example, it is contemplated that executing or performing a particular operation before, contemporaneously with, or after another operation is within the scope of aspects of the invention.

Embodiments may be implemented with computer-executable instructions. The computer-executable instructions may be organized into one or more computer-executable components or modules. Aspects of the disclosure may be implemented with any number and organization of such components or modules. For example, aspects of the disclosure are not limited to the specific computer-executable instructions or the specific components or modules illustrated in the figures and/or described herein. Other embodiments may include different computer-executable instructions or components having more or less functionality than illustrated and described herein.

Many other embodiments of the present disclosure are also envisioned. For example, a method comprises determining a person's level of risk for developing an adverse condition; selecting a care protocol based on the level of risk; displaying a proposed configuration of a person support structure corresponding to the care protocol for a caregiver to approve; and upon approval by the caregiver, implementing the configuration. In another contemplated embodiment, the configuration causes the person support structure to raise a first section of the person support structure such that the first section forms an angle of greater than 0° with respect to the reference plane. In another contemplated embodiment, the configuration causes the person support structure to laterally tilt an occupant supported on the person support structure to a side such that the occupant is are at an angle of greater than 0° with respect to the reference plane. In another contemplated embodiment, the configuration causes the person support structure to move to at least one of a Trendelenburg and reverse Trendelenburg position. In another contemplated embodiment, the configuration causes a therapy to be initiated. In another contemplated embodiment, the therapy includes heat and moisture regulating therapy. In another contemplated embodiment, the therapy includes continuous lateral rotation therapy. In another contemplated embodiment, the therapy includes at least one of percussion therapy and vibration therapy. In another contemplated embodiment, the proposed configuration is modified as a function of a second input indicative of the orientation of a person supported on the person support structure. In another contemplated embodiment, the proposed configuration is modified as a function of a second input indicative of the position of a person supported on the person support structure. In another contemplated embodiment, the method further comprises the steps of: receiving an input indicative of the sleep state of the person supported on the person support structure; and if the person is waking up, restoring the person support structure to a previous configuration. In another contemplated embodiment, the person support structure is configured upon an occupant reaching a predetermined sleep stage. In another contemplated embodiment, the method further comprises the steps of: receiving a configuration override command, and restoring the person support structure to a previous configuration. In one contemplated embodiment, the configuration override command is communicated from a remote location. In another contemplated embodiment, the configuration override command is communicated when a CPR function is activated. In another contemplated embodiment, the configuration override command is communicated from a GUI coupled to the person support structure. In another contemplated embodiment, the method further comprises the step of notifying a caregiver if the presence of a material would aggravate an adverse condition. In another contemplated embodiment, the method further comprises the steps of: receiving an input indicative of a material proximate to the person supported on the person support structure determining if the material increases the person's risk for developing an adverse condition.

In another example, a method comprises receiving a signal indicative of a physiological characteristic; comparing the signal to a threshold to determine if an adverse event is in progress; and upon detecting that an adverse event is in progress, initiating an intervention to stop the adverse event. In one contemplated embodiment, the second intervention includes increasing the magnitude of the first intervention. In another contemplated embodiment, the second intervention includes alerting a caregiver.

In another example, a person support surface comprises a mattress ticking and a mattress core. The mattress core is enclosed by the mattress ticking and includes at least one fluid bladder configured to selectively protrude from the person contacting surface and support a portion of at least one of the neck and the upper back of an occupant supported on the person support surface. In one contemplated embodiment, the at least one fluid bladder is configured to support the cervical vertebrae of an occupant. In another contemplated embodiment, the at least one fluid bladder is configured to protrude a distance of at least about 70mm from the occupant facing surface. In another contemplated embodiment, the at least one fluid bladder is configured to support the scapula of air occupant. In another contemplated embodiment, the at least one fluid bladder is configured to protrude a distance of at least about 20mm from the occupant facing surface. In another contemplated embodiment, the at least one fluid bladder is configured to protrude a distance of about 20mm to about 30mm from the occupant facing surface. In another contemplated embodiment, the at least one fluid bladder is configured to protrude a distance of less than about 30mm from the occupant facing surface. In another contemplated embodiment, the at least one fluid bladder is configured to laterally tilt an occupant's head when inflated. In another contemplated embodiment, the at least one fluid bladder is inflated upon detecting the onset of an adverse condition.

In another example, a method comprises determining a person's level of risk for developing an adverse condition; selecting a care protocol based on the level of risk; sensing a first physiological characteristic of a person supported on a person support structure; sensing a second physiological characteristic of the person; comparing the first physiological characteristic to the second physiological characteristic; if the difference between the first physiological characteristic and second physiological characteristic is outside a predefined range, configuring the person support structure as a function of the care protocol.

In another example, a method comprises determining a person's level of risk for developing an adverse condition; selecting a care protocol based on the level of risk; sensing a first physiological characteristic of a person supported on a person support structure; sensing a second physiological characteristic of the person; comparing the first physiological characteristic to the second physiological characteristic; if the difference between the first physiological characteristic and second physiological characteristic is outside a predefined range, alerting a caregiver that an adverse condition is going to occur.

Any theory, mechanism of operation, proof, or finding stated herein is meant to further enhance understanding of principles of the present disclosure and is not intended to make the present disclosure in any way dependent upon such theory, mechanism of operation, illustrative embodiment, proof, or finding. It should be understood that while the use of the word preferable, preferably or preferred in the description above indicates that the feature so described can be more desirable, it nonetheless cannot be necessary and embodiments lacking the same can be contemplated.

While embodiments of the disclosure have been illustrated and described in detail in the drawings and foregoing description, the same are to be considered as illustrative and not intended to be exhaustive or to limit the disclosure to the precise forms disclosed. Additional alternatives, modifications and variations can be apparent to those skilled in the art, whereby the invention is solely defined by the appended claims.

## Claims

1. A support system configured to support and laterally rotate at least a portion of a user with respect to a longitudinal axis of the support system, said support system comprising a plurality of support pieces (1102, 1106, 1110, 1162, 1166, 1170, 1312) configured to collectively define a laterally angled sleep surface (1114) having a length defined between a first edge and an opposing second edge spaced from the first edge along the longitudinal axis, a first support piece of the plurality of support pieces (1102, 1106, 1110, 1162, 1166, 1170, 1312) forming a first support plane along the longitudinal axis having a first angle of rotation with respect to a base surface of the support system and a second support piece of the plurality of support pieces (1102, 1106, 1110, 1162, 1166, 1170, 1312) forming a second support plane along the longitudinal axis having a second angle of rotation with respect to the base surface different than the first angle of rotation, **characterized in that**:
the plurality of support pieces (1102, 1106, 1110, 1162, 1166, 1170, 1312) collectively define a segmented, multi-plane, laterally angled sleep surface (1114) having progressively greater angles of rotation along a longitudinal axis of the support system, from the first edge of the sleep surface (1114) to the second edge of the sleep surface (1114), resulting in relatively greater rotation of the upper respiratory tract of the user and relatively lesser rotation in the lower body of the user

2. The support system of claim 1, wherein at least one support piece is rotatable about the longitudinal axis.

3. The support system of any preceding claim, wherein the second angle of rotation is greater than the first angle of rotation to facilitate greater rotation of an upper respiratory tract of the user supported on the second support plane than a lower body of the user at least partially supported on the first support plane.

4. The support system of any preceding claim, wherein each support piece of the plurality of support pieces is independently rotatable about an axis extending parallel with the longitudinal axis of the support system.

5. The support system of any preceding claim, wherein the first support plane is positioned at a first lateral angle of rotation of 20° to 30° with respect to a base surface of the first support piece, the second support plane is positioned at a second lateral angle of rotation of 10° to 20° with respect to a base surface of the second support piece, and a third support piece defines a third support surface positioned at a third lateral angle of rotation of 5° to 15° with respect to a base surface of the third support piece.

6. The support system of any preceding claim, further comprising a transitional piece (1130, 1132) positioned between adjacent support pieces of the plurality of support pieces (1102, 1106, 1110, 1162, 1166, 1170, 1312) to facilitate providing a gradual continuous transition between support planes of the adjacent support pieces.

7. The support system of claim 6 wherein the transitional piece (1130, 1132) is positioned at a transitional line where the first support piece meets with the second support piece to provide support for the user.

8. The support system of any preceding claim, further comprising a system control (1190, 1302) having a display configured to display information about the support system, wherein preferably the system control comprises a processor configured to adjust angles of rotation of the support planes based at least in part on data inputted at the system control and/or data signals received from one or more sensors of the support system.

9. The support system of any preceding claim, further comprising a bolster (1142, 1176) positioned along a lateral side of the support system to limit lateral migration of the user, wherein preferably at least a portion of the bolster (1142, 1176) includes a textured surface to facilitate retaining the user positioned on the sleep surface, wherein preferably the bolster (1142, 1176) is formed of a formable material to provide envelopment throughout the sleep surface, and wherein preferably the support system further comprises a strap operatively coupled to the bolster (1142, 1176) to facilitate maintaining the user positioned on the sleep surface.

10. The support system of any preceding claim, wherein each support piece of the plurality of support pieces (1102, 1106, 1110, 1162, 1166, 1170, 1312) is rotatable about the longitudinal axis between a first orientation having a right side slope and a second orientation having a left side slope, and the support system preferably further comprises a section positioned within the first support piece and coaxially aligned with the longitudinal axis to facilitate rotation of the first support piece about the longitudinal axis independently of the second support piece.

11. The support system of any preceding claim, further comprising at least one spacer (1150) configured to adjust a length of at least one support piece of the support surface.

12. The support system of any preceding claim, wherein at least one support piece comprises an inflatable fluid bladder (1162, 1166, 1170, 1312) configured to contain a fluid, and wherein preferably an amount of fluid within the fluid bladder (1162, 1166, 1170, 1312) is controlled electronically, and wherein the support system preferably further comprises a coupler configured to at least one of to maintain an amount of fluid within the fluid bladder (1162, 1166, 1170, 1312) and provide support to the respective support plane.

13. The support system of any preceding claim, wherein the first support piece (1102) comprises a plurality of independent support wedges forming a gradation in a longitudinal slope of the sleep surface, and wherein preferably each of the plurality of independent support wedges comprises one of a formable material, a semi-rigid material, a foam material, and a fluid bladder, and wherein preferably a first support wedge of the plurality of independent support wedges defines a first support plane positioned at a first lateral angle of rotation and a second support wedge of the plurality of independent support wedges defines a second support plane at a second lateral angle of rotation different that the first lateral angle of rotation.

## Patentansprüche

1. Ein Unterstützungssystem, das so konfiguriert ist, dass es mindestens einen Teil eines Benutzers in Bezug auf eine Längsachse des Unterstützungssystems unterstützt und seitwärts dreht, wobei das genannte Unterstützungssystem eine Vielzahl an Unterstützungsstücken (1102, 1106, 1110, 1162, 1166, 1170, 1312) umfasst, die so konfiguriert sind, dass sie insgesamt eine seitwärts abgewinkelte Liegefläche (1114) mit einer Länge definieren, die zwischen einer ersten Kante und einer gegenüberliegenden zweiten Kante, die im Abstand von der ersten Kante entlang der Längsachse angeordnet ist, definiert ist, wobei ein erstes Unterstützungsstück von der Vielzahl an Unterstützungsstücken (1102, 1106, 1110, 1162, 1166, 1170, 1312) eine erste Unterstützungsebene entlang der Längsachse mit einem ersten Drehwinkel in Bezug auf eine Grundfläche des Unterstützungssystems und ein zweites Unterstützungsstück von der Vielzahl an Unterstützungsstücken (1102, 1106, 1110, 1162, 1166, 1170, 1312) eine zweite Unterstützungsebene entlang der Längsachse mit einem zweiten Drehwinkel in Bezug auf die Grundfläche, der sich von dem ersten Drehwinkel unterscheidet, bildet, **dadurch gekennzeichnet, dass**:
die Vielzahl an Unterstützungsstücken (1102, 1106, 1110, 1162, 1166, 1170, 1312) insgesamt eine segmentierte, multiplanare, seitwärts abgewinkelte Liegefläche (1114) mit zunehmend größeren Drehwinkeln entlang einer Längsachse des Unterstützungssystems definiert, von der ersten Kante der Liegefläche (1114) zur zweiten Kante der Liegefläche (1114), was zu einer relativ größeren Drehung des oberen Respirationstraktes des Benutzers und zu einer relativ geringeren Drehung des Unterkörpers des Benutzers führt.

2. Das Unterstützungssystem nach Anspruch 1, wobei mindestens ein Unterstützungsstück um die Längsachse drehbar ist.

3. Das Unterstützungssystem nach einem der vorstehenden Ansprüche, wobei der zweite Drehwinkel größer als der erste Drehwinkel ist, um eine größere Drehung des von der zweiten Unterstützungsebene gestützten oberen Respirationstraktes eines Benutzers als des mindestens teilweise von der ersten Unterstützungsebene gestützten Unterkörpers eines Benutzers zu ermöglichen.

4. Das Unterstützungssystem nach einem der vorstehenden Ansprüche, wobei jedes Unterstützungsstück von der Vielzahl an Unterstützungsstücken unabhängig um eine Achse drehbar ist, die parallel zur Längsachse des Unterstützungssystems verläuft.

5. Das Unterstützungssystem nach einem der vorstehenden Ansprüche, wobei sich die erste Unterstützungsebene in einem ersten seitlichen Drehwinkel von 20° bis 30° in Bezug auf eine Grundfläche des ersten Unterstützungsstücks befindet, sich die zweite Unterstützungsebene in einem zweiten seitlichen Drehwinkel von 10° bis 20° in Bezug auf eine Grundfläche des zweiten Unterstützungsstücks befindet, und ein drittes Unterstützungsstück eine dritte Unterstützungsfläche definiert, die sich in einem dritten seitlichen Drehwinkel von 5° bis 15° in Bezug auf eine Grundfläche des dritten Unterstützungsstücks befindet.

6. Das Unterstützungssystem nach einem der vorstehenden Ansprüche, das weiter ein Übergangsstück (1130, 1132) umfasst, das sich zwischen benachbarten Unterstützungsstücken von der Vielzahl an Unterstützungsstücken (1102, 1106, 1110, 1162, 1166, 1170, 1312) befindet, um die Bereitstellung eines schrittweisen kontinuierlichen Übergangs zwischen den Unterstützungsebenen von benachbarten Unterstützungsstücken zu ermöglichen.

7. Das Unterstützungssystem nach Anspruch 6, wobei sich das Übergangsstück (1130, 1132) an einer Übergangslinie befindet, wo das erste Unterstützungsstück auf das zweite Unterstützungsstück trifft, um Unterstützung für den Benutzer bereitzustellen.

8. Das Unterstützungssystem nach einem der vorstehenden Ansprüche, weiter umfassend eine Systemsteuerung (1190, 1302) mit einer Anzeige, die so konfiguriert ist, dass Informationen über das Unterstützungssystem angezeigt werden, wobei die Systemsteuerung vorzugsweise einen Prozessor umfasst, der so konfiguriert ist, dass die Drehwinkel der Unterstützungsebenen zumindest teilweise basierend auf den in die Systemsteuerung eingegebenen Daten und/oder den von einem oder mehreren Sensoren des Unterstützungssystems empfangenen Daten eingestellt werden.

9. Das Unterstützungssystem nach einem der vorstehenden Ansprüche, weiter umfassend ein Polster (1142, 1176), das sich entlang einer lateralen Seite des Unterstützungssystems befindet, um die seitliche Wanderungsbewegung des Benutzers zu begrenzen, wobei vorzugsweise mindestens ein Teil des Polsters (1142, 1176) eine strukturierte Oberfläche beinhaltet, damit der Benutzer auf der Liegefläche gehalten werden kann, wobei vorzugsweise das Polster (1142, 1176) aus einem verformbaren Material gebildet ist, um eine Umhüllung durch die Liegefläche bereitzustellen, und wobei vorzugsweise das Unterstützungssystem weiter einen Riemen umfasst, der einsatzbereit an das Polster (1142, 1176) gekoppelt ist, damit der Benutzer auf der Liegefläche in Position gehalten werden kann.

10. Das Unterstützungssystem nach einem der vorstehenden Ansprüche, wobei jedes Unterstützungsstück von der Vielzahl an Unterstützungsstücken (1102, 1106, 1110, 1162, 1166, 1170, 1312) um die Längsachse zwischen einer ersten Ausrichtung mit einer Neigung zur rechten Seite und einer zweiten Ausrichtung mit einer Neigung zur linken Seite drehbar ist, und das Unterstützungssystem vorzugsweise weiter einen Abschnitt umfasst, der sich innerhalb des ersten Unterstützungsstücks befindet und koaxial mit der Längsachse ausgerichtet ist, um eine Drehung des ersten Unterstützungsstücks um die Längsachse unabhängig von dem zweiten Unterstützungsstück zu ermöglichen.

11. Das Unterstützungssystem nach einem der vorstehenden Ansprüche, weiter umfassend mindestens einen Abstandhalter (1150), der so konfiguriert ist, dass eine Länge des mindestens einen Unterstützungsstücks der Liegefläche angepasst wird.

12. Das Unterstützungssystem nach einem der vorstehenden Ansprüche, wobei mindestens ein Unterstützungsstück eine befüllbare Flüssigkeitsblase (1162, 1166, 1170, 1312) umfasst, die so konfiguriert ist, dass sie eine Flüssigkeit enthält, und wobei vorzugsweise eine Flüssigkeitsmenge innerhalb der Flüssigkeitsblase (1162, 1166, 1170, 1312) elektronisch gesteuert wird, und wobei das Unterstützungssystem vorzugsweise weiter ein Kopplungsstück umfasst, das so konfiguriert ist, dass mindestens eine Flüssigkeitsmenge innerhalb der Flüssigkeitsblase (1162, 1166, 1170, 1312) gehalten wird und Unterstützung für die jeweilige Unterstützungsebene bereitstellt.

13. Das Unterstützungssystem nach einem der vorstehenden Ansprüche, wobei das erste Unterstützungsstück (1102) eine Vielzahl an unabhängigen Unterstützungskeilen umfasst, die eine Abstufung in einer Längsneigung der Liegefläche bilden, und wobei vorzugsweise jeder der Vielzahl an unabhängigen Unterstützungskeilen eines von einem verformbaren Material, einem halbstarren Material, einem Schaummaterial und einer Flüssigkeitsblase umfasst, und wobei vorzugsweise ein erster Unterstützungskeil von der Vielzahl an unabhängigen Unterstützungskeilen eine erste Unterstützungsebene, die sich in einem ersten seitlichen Drehwinkel befindet, definiert und ein zweiter Unterstützungskeil von der Vielzahl an unabhängigen Unterstützungskeilen eine zweite Unterstützungsebene, die sich in einem zweiten seitlichen Drehwinkel, der sich vom ersten seitlichen Drehwinkel unterscheidet, befindet, definiert.

## Revendications

1. Système de soutien conçu pour soutenir et faire tourner latéralement au moins une partie d'un utilisateur par rapport à un axe longitudinal du système de soutien, ledit système de soutien comportant une pluralité d'éléments de soutien (1102, 1106, 1110, 1162, 1166, 1170, 1312) conçus pour délimiter ensemble une surface de sommeil (1114) inclinée latéralement présentant une longueur définie entre un premier bord et un second bord opposé espacé du premier bord le long de l'axe longitudinal, un premier élément de soutien de la pluralité d'éléments de soutien (1102, 1106, 1110, 1162, 1166, 1170, 1312) formant un premier plan de soutien le long de l'axe longitudinal possédant un premier angle de rotation par rapport à une surface de base du système de soutien et un deuxième élément de soutien de la pluralité d'éléments de soutien (1102, 1106, 1110, 1162, 1166, 1170, 1312) formant un second plan de soutien le long de l'axe longitudinal possédant un deuxième angle de rotation par rapport à la surface de base différent du premier angle de rotation, **caractérisé en ce que** :
la pluralitsoutien conçu pour soutenir et faire tourner latéralement au moins une part ensemble une surface de sommeil (1114) inclinée latéralement, à plans multiples et segmentée possédant des angles de rotation progressivement plus grands le long d'un axe longitudinal du système de soutien, depuis le premier bord de la surface de sommeil (1114) jusqu'au second bord de la surface de sommeil (1114), ce qui permet une rotation relativement plus grande des voies respiratoires supérieures de l'utilisateur et une rotation relativement plus petite de la partie inférieure du corps de l'utilisateur.

2. Système de soutien selon la revendication 1, dans lequel au moins un élément de soutien peut tourner autour de l'axe longitudinal.

3. Système de soutien selon l'une quelconque des revendications précédentes, dans lequel le deuxième angle de rotation est plus grand que le premier angle de rotation pour faciliter une plus grande rotation des voies respiratoires supérieures de l'utilisateur soutenu sur le second plan de soutien par rapport à une partie inférieure du corps de l'utilisateur au moins partiellement soutenu sur le premier plan de soutien.

4. Système de soutien selon l'une quelconque des revendications précédentes, dans lequel chaque élément de soutien de la pluralité d'éléments de soutien peut tourner indépendamment autour d'un axe s'étendant parallèlement à l'axe longitudinal du système de soutien.

5. Système de soutien selon l'une quelconque des revendications précédentes, dans lequel le premier plan de soutien est placé selon un premier angle de rotation latérale de 20° à 30° par rapport à une surface de base du premier élément de soutien, le deuxième plan de soutien est placé selon un deuxième angle de rotation latérale de 10° à 20° par rapport à une surface de base du deuxième élément de soutien, et un troisième élément de soutien délimite une troisième surface de soutien placée selon un troisième angle de rotation latérale de 5° à 15° par rapport à une surface de base du troisième élément de soutien.

6. Système de soutien selon l'une quelconque des revendications précédentes, comprenant en outre un élément de transition (1130, 1132) placé entre les éléments de soutien adjacents de la pluralité d'éléments de soutien (1102, 1106, 1110, 1162, 1166, 1170, 1312) pour faciliter la fourniture d'une transition continue progressive entre les plans de soutien des éléments de soutien adjacents.

7. Système de soutien selon la revendication 6, dans lequel l'élément de transition (1130, 1132) est placé au niveau d'une ligne de transition où le premier élément de soutien rencontre le deuxième élément de soutien pour fournir un soutien destiné à l'utilisateur.

8. Système de soutien selon l'une quelconque des revendications précédentes, comprenant en outre une commande de système (1190, 1302) possédant un écran conçu pour afficher des informations concernant le système de soutien, la commande de système comprenant de préférence un processeur conçu pour régler les angles de rotation des plans de soutien sur la base, au moins en partie, des données entrées au niveau de la commande de système et/ou des signaux de données reçus en provenance d'un ou de plusieurs capteurs du système de soutien.

9. Système de soutien selon l'une quelconque des revendications précédentes, comprenant en outre un traversin (1142, 1176) placé le long d'un côté latéral du système de soutien pour limiter le déplacement latéral de l'utilisateur, dans lequel de préférence au moins une partie du traversin (1142, 1176) comprend une surface texturée permettant de faciliter le maintien de l'utilisateur placé sur la surface de sommeil, le traversin (1142, 1176) étant de préférence constitué d'un matériau malléable pour fournir l'enveloppement d'un bout à l'autre de la surface de sommeil, et le système de soutien comprenant en outre de préférence une sangle couplée de manière fonctionnelle au traversin (1142, 1176) pour faciliter le maintien de l'utilisateur placé sur la surface de sommeil.

10. Système de soutien selon l'une quelconque des revendications précédentes, dans lequel chaque élément de soutien de la pluralité d'éléments de soutien (1102, 1106, 1110, 1162, 1166, 1170, 1312) peut tourner autour de l'axe longitudinal entre une première orientation présentant une pente côté droit et une seconde orientation présentant une pente côté gauche, et le système de soutien comprend en outre de préférence une section placée à l'intérieur du premier élément de soutien et alignée de manière coaxiale avec l'axe longitudinal pour faciliter la rotation du premier élément de soutien autour de l'axe longitudinal indépendamment du deuxième élément de soutien.

11. Système de soutien selon l'une quelconque des revendications précédentes, comprenant en outre au moins un élément d'espacement (1150) conçu pour ajuster une longueur d'au moins un élément de soutien de la surface de soutien.

12. Système de soutien selon l'une quelconque des revendications précédentes, dans lequel au moins un élément de soutien comprend une poche de fluide gonflable (1162, 1166, 1170, 1312) conçue pour contenir un fluide, et de préférence une quantité de fluide à l'intérieur de la poche de fluide (1162, 1166, 1170, 1312) étant régulée par voie électronique, et le système de soutien comprenant en outre de préférence un élément d'accouplement conçu pour au moins pour maintenir une quantité de fluide à l'intérieur de la poche de fluide (1162, 1166, 1170, 1312) ou pour fournir un soutien au plan de soutien respectif.

13. Système de soutien selon l'une quelconque des revendications précédentes, dans lequel le premier élément de soutien (1102) comprend une pluralité de cales de soutien indépendantes formant une gradation selon une pente longitudinale de la surface de sommeil, et de préférence chacune de la pluralité de cales de soutien indépendantes comprend un élément parmi un matériau malléable, un matériau semi-rigide, un matériau en mousse, et une poche de fluide, et de préférence une première cale de soutien de la pluralité des cales de soutien indépendantes délimite un premier plan de soutien placé selon un premier angle de rotation latérale et une seconde cale de soutien de la pluralité de cales de soutien indépendantes délimite un second plan de soutien selon un deuxième angle de rotation latérale différent du premier angle de rotation latérale.
